# EUROPEAN PATENT APPLICATION

(11) **EP 1 419 785 A1**
(43) Date of publication of application: **19.05.2004**
(21) Application number: 02760743.1
(22) Date of filing: 26.08.2002
(51) Int. Cl.: A61K 45/06, A61K 31/4184, A61P 9/00, A61P 13/12

(54) **DRUGS CONTAINING CHYMASE INHIBITOR AND ACE INHIBITOR AS THE ACTIVE INGREDIENTS**

(30) Priority: 24.08.2001 JP 2001254120
(71) Applicant: TEIJIN LIMITED, Osaka-shi Osaka 541-0054 (JP)
(72) Inventor: URATA, H., c/o Fukuoka Univ. Chikushi Hospital, Chikushino-shi, Fukuoka 818-0067 (JP); HASE, N., c/o Teijin Ltd,Tokyo Research Center, Hino-shi, Tokyo 191-0065 (JP); TSUCHIYA, N., c/o Teijin Ltd,Tokyo ResearchCenter, Hino-shi, Tokyo 191-0065 (JP)
(74) Representative: Hallybone, Huw George
(86) International application number: PCT/JP2002/008572
(87) International publication number: WO 2003/018061

(57) **Abstract**

It is an object of the present invention to provide drugs which are effective for treatment of hypertension, cardiac disease (cardiac hypertrophy, cardiac failure, myocardial infarction, etc.), cerebral apoplexy, nephritis and the like. The invention also provides circulatory disease treatment agents in forms that allow combined use of chymase inhibitors and ACE inhibitors, and circulatory disease treatment methods which produce simultaneous chymase inhibition and ACE inhibition.

## Description

### Technical Field

The present invention relates to drugs comprising both chymase inhibitors and ACE (Angiotensin-Converting Enzyme) inhibitors as effective ingredients, and to prophylactic or treatment methods involving administration of the chymase inhibitors and ACE inhibitors. More specifically, the invention relates to the drugs or methods as prophylactic or treatment agents and prophylactic or treatment methods for circulatory diseases, or to angiotensin II-production suppressors and a suppressing method. The drugs of the invention exhibit a powerful angiotensin II-production suppressing effect and are therefore effective for treatment of hypertension, cardiac disease (cardiac hypertrophy, cardiac failure, myocardial infarction, etc.), cerebral apoplexy, nephritis and the like.

### Background Art

Angiotensin II has been extensively studied as a major factor in the renin-angiotensin system which plays an important role in maintaining bodily homeostasis including systemic blood pressure and body fluid volume. The powerful vasoconstricting effect of angiotensin II has made it the major object of attention as a causative substance in hypertensive disorders, and drug therapies for circulatory diseases have been developed which suppress its function. In recent years, angiotensin II has been found to act as a growth factor promoting growth of fibroblasts and has been associated with regulation of a wide variety of cellular functions, particularly in relation to cardiovascular conditions, including cardiac muscle cell hypertrophy, smooth muscle cell migration and growth, stimulation of fibroblast intracellular matrix production and inducement of apoptosis, while it is also thought to play an important role in formation of fibrosis and renal sclerotic and arteriosclerotic lesions. Inhibitors of angiotensin-converting enzyme (hereinafter abbreviated as "ACE"), the enzyme which produces angiotensin II, have long been used as the first agents of choice for circulatory diseases, and their effectiveness has been clearly demonstrated in the clinic.

In the course of elucidating the importance of tissue local angiotensin II production systems, there has been discovered an ACE-independent pathway for local production of angiotensin II in human tissue, and in particular, an angiotensin II production pathway involving human mast cell chymase has attracted considerable attention. For example, ACE inhibitors have been shown to be ineffective for restenosis after Percutaneous Transluminal Coronary Angioplasty (PTCA) (Circulation 1992; 86:100-110, J. Am. Coll. Cardiol 1995; 25:362-369), suggesting the existence of an enzyme other than ACE for production of angiotensin II. Urata et al. have isolated and extracted from human heart an enzyme that produces angiotensin II from angiotensin I and, based on the chemical structure and gene cloning of the enzyme, have determined that human mast cell chymase performs an angiotensin II-producing role (J. Biol. Chem 1990; 265: 222348-22357, J. Biol. Chem. 1991; 266: 17173-17179). Research to date has confirmed the presence of chymase only in tissue connective mast cells, from which it is considered to carry out a physiological function only in local tissue. In the vascular walls, chymase is normally most abundant in the adventitia while ACE is localized in the intima, but it is believed that chymase is involved in reconstruction and regenerative intima growth in the case of vascular injury (Circulation 1996; 94: 1655-1664).

The ACE-independent angiotensin II production pathway involving chymase has been substantiated by numerous other reports indicating its greater contribution in humans compared to other animal species. Since the vasoconstrictive effect by addition of angiotensin I in, for example, simian and canine extracted vessels had long been reported to be suppressed only by about 30% with ACE inhibitors alone, while complete suppression was only achieved by a combined effect with chymostatin, a serine protease-inhibiting protein, this led to conjecture regarding the existence of an angiotensin II production pathway involving an enzyme other than ACE, and the contribution of such an ACE-independent angiotensin II production pathway was proposed to be more notable in larger animals than in rodents (J. Hypertensions 1984; 2: 277-189). This ACE-independent angiotensin II production pathway was later shown to involve chymase, while other reports indicated that chymase carries out approximately 80% of the production of angiotensin II in human hearts (Circ. Res. 1990; 66: 883-890).

Angiotensin II receptor antagonists have recently been developed for humans in order to achieve simultaneous suppression of the two major angiotensin II production pathways, ACE-dependent and ACE-independent. Since angiotensin II acts through cell membrane-bound angiotensin II receptors, much of the development has been aimed at antagonists of the AT1 receptor, which is the angiotensin II receptor associated with vasoconstriction (Japanese Unexamined Patent Publication SHO No. 56-17073, EP0253310, EP0291969, EP0324377, Japanese Unexamined Patent Publication SHO No. 63-23868, Japanese Unexamined Patent Publication HEI No. 1-1178676, EP0323841, Japanese Unexamined Patent Publication HEI No. 1-287071, Japanese Unexamined Patent Publication HEI No. 4-364171, and others). Most of these receptor antagonists have come into clinical use as antihypertensive agents, and their effects have been recognized as being equal to or greater than those of ACE inhibitors. The clinical utility of suppressing both ACE-dependent and ACE-independent angiotensin II with these receptor antagonists has also been verified. Nevertheless, not all of the problems arising with treatment of circulatory diseases using angiotensin II receptor antagonists have been solved.

Large-scale clinical trials of AT1 receptor antagonists are being conducted and their clinical effects are gradually coming to light. However, while the effects of AT1 receptor antagonists improve patient QOL (Quality of Life), particularly in the case of cardiac diseases such as heart failure, with respect to hospital readmission due to recurring cardiovascular events and aggravated heart failure, the actual reduction in overall mortality of heart failure patients is approximately the same as with ACE inhibitors, such that satisfactory results have not yet been achieved for treatment of cardiac disease (ELITE II Trial, Lancet 2000, Vol.355 p.1582, Annual Meeting of the American Heart Society, 2000, 2001; New England Journal of Medicine 2001, Vol.345, p.1667, Annual Meeting of the American Heart Society, 2000, 2001).

Recent reports of research with knockout mice have suggested that angiotensin II may act as an aggravating factor through the angiotensin II receptor AT2, in conditions of cardiac hypertrophy and fibrosis (Circulation, 2001, Vol.104, p.247, Ichihara et al.; Trends Cardiovasc. Med., 2001, Vol.11, p.324, Inagami et al.).

Moreover, some hypertensive patients with circulatory diseases other than cardiac disease do not experience lowered blood pressure even with ACE inhibitors or angiotensin II receptor antagonists. The therapeutic effects of ACE inhibitors and angiotensin II receptor antagonists for pulmonary hypertension have not been determined.

The present inventors have therefore developed treatment agents and a treatment method based on a novel concept not used in the clinic to date, and have proposed the present invention as a solution to the problems associated with current treatment of circulatory diseases.

### Disclosure of the Invention

It is an object of the present invention to provide novel treatment agents for hypertension-associated cardiovascular disease, including cardiac disease (cardiac hypertrophy, cardiac failure, myocardial infarction, etc.), cerebral apoplexy, vascular injury such as post-PTCA restenosis, arteriosclerosis, renal failure, nephritis, some types of hypertension, and pulmonary hypertension.

The following aspects may be considered to explain why, even though AT1 receptor antagonists simultaneously suppress both ACE-dependent and ACE-independent angiotensin II activity, the expected treatment effect has not been achieved for cardiac diseases. Namely, these receptor antagonists are AT1 receptor-selective antagonists and do not suppress angiotensin II receptors other than AT1 receptors. In fact, since antagonism of AT1 receptors is known to raise angiotensin II concentrations in the blood and tissues, the increased angiotensin II could be stimulating other receptors. For example, the angiotensin II receptor AT2 is thought to have an important function similar to that of AT1 receptor, but its role in circulatory disease is still incompletely understood.

The aforementioned report suggesting that angiotensin II may act as an aggravating factor through the AT2 receptor in conditions of cardiac hypertrophy and fibrosis partially supports the theory according to which increased angiotensin II concentrations in the blood and tissues due to AT1 receptor antagonism, leading to angiotensin II stimulation of other receptors, results in aggravation of circulatory disease conditions. Consequently, while cardiac diseases including chronic cardiac failure eventually lead to death by diffuse cardiac fibrosis and accompanying reduced cardiac function, it is possible that AT2 receptor stimulation during progression of such cardiac conditions may be involved in their aggravation. Such AT2 receptor stimulation could be the reason for failure to achieve a sufficient therapeutic effect for cardiac diseases such as chronic heart failure, cardiac dysfunction following myocardial infarction, etc. when using AT1 receptor antagonists.

ACE inhibitors which are widely used for clinical treatment of circulatory diseases are thought to also exhibit a therapeutic effect on circulatory diseases by inhibiting decomposition of bradykinin, etc., and for this reason angiotensin receptor antagonists cannot fully substitute for the therapeutic effects of ACE inhibitors.

In addition, since chymase has powerful vasoconstricting action like angiotensin II and activates endothelin which has proliferating action on various cell types, it may participate in the pathology of circulatory diseases based on mechanisms not involving angiotensin II receptors. Chymase is believed to aggravate inflammatory reaction at sites of cardiovascular injury through activation of the inflammatory cytokine IL-1β, to contribute to decomposition of fibronectin and type IV collagen and decomposition of extracellular proteins through activation of matrix proteases, and to accelerate differentiation and growth of fibroblasts and thus promote tissue fibrosis/tissue remodeling, by promoting release of transforming growth factor β (TGFβ). Thus, chymase inhibitors are considered to be useful therapeutic agents for such circulatory diseases by not only inhibiting angiotensin II function but also exhibiting a wide range of pharmacological effects for which angiotensin receptor antagonists cannot substitute. Incidentally, benzimidazole derivatives and their medically acceptable salts have been disclosed as chymase inhibitors (WO01/53291, WO01/53272, WO00/03997). Their chemical structure will be described below.

The present inventors have come to believe that treatment methods and agents applying simultaneous administration of ACE inhibitors and chymase inhibitors will constitute revolutionary treatment methods and agents for circulatory diseases whereby a synergistic therapeutic effect may be expected. Therefore, the present invention introduces the concept of novel treatment agents for circulatory diseases, which have effects of not only inhibiting ACE-dependent and ACE-independent angiotensin II production in tissue, but also of exhibiting the ACE inhibitor effect of suppressing bradykinin decomposition, which is not exhibited by AT1 receptor antagonists, as well as the effects of chymase, including those of suppressing interleukin-1β (IL-1β) activation, matrix protease activation, fibronectin and type IV collagen decomposition, transforming growth factor-β (TGF-β) release, and substance P and vasoactive intestinal peptide (VIP) activation.

It is believed that ACE and chymase perform their angiotensin II production-related roles at local pathological sites, and that chymase is more crucial than ACE in vascular injury conditions such as post-PTCA restenosis. In addition, reports indicating increased chymase activity at sites of vascular pulp arteriosclerosis, a role for chymase in promoting pulmonary vascular lesions in congenital cardiac disease, and chymase induced cardiac muscle cell apoptosis and cardiac fibroblast growth, have implicated chymase-mediated angiotensin II production as carrying out a role related to but distinct from that of ACE in circulatory tissue remodeling of primarily injured vessels and the heart.

It is therefore believed that simultaneously inhibiting both the ACE-mediated angiotensin II production and chymase-mediated angiotensin II production pathways to reduce actual angiotensin II levels in the body will be effective for treatment of circulatory diseases, and will thus constitute effective means of therapy for cardiac failure, myocardial infarction and its prognosis, post-PCTA vascular restenosis, cardiac conditions accompanying pulmonary hypertension, arteriosclerosis, renal failure and some forms of hypertension.

The present inventors have conducted much diligent research focused on the close relationship between chymase and ACE through angiotensin II production in the context of cardiovascular disease. Homologs of human chymase have been identified in rodent species such as mice and rats, but these homologous enzymes, unlike the human enzyme, have both angiotensin II-producing activity and angiotensin II-degrading activity, and the contribution of the chymase-dependent angiotensin II production pathway is thus considered less important in rodents. Hamsters, whose chymase-mediated angiotensin II production is relatively closer to that of humans, have been used as experimental animals in order to study the contributory role of the chymase angiotensin II production pathway. The human chymase gene has also been transferred to mouse zygotes to create human chymase-overexpressing mice, and these human chymase-producing transgenic animals have been used to study the effects on circulatory diseases.

Based on these advanced research results, the present inventors have discovered that simultaneous administration of chymase inhibitors and ACE inhibitors can constitute highly effective treatment for circulatory diseases.

In other words, the invention relates to drugs comprising chymase inhibitors and ACE inhibitors as effective ingredients, and to prophylactic or treatment methods involving administration of the chymase inhibitors and ACE inhibitors.

The invention more specifically relates to prophylactic or treatment agents for circulatory diseases comprising chymase inhibitors and ACE inhibitors as effective ingredients, and to prophylactic or treatment methods for circulatory diseases involving administration of the chymase inhibitors and ACE inhibitors,

The invention still further relates to such treatment agents or treatment methods wherein the particular target circulatory disease is hypertension, cardiac disease, cerebral apoplexy, vascular injury, arteriosclerosis, nephritis or renal failure.

The invention still further relates to such treatment agents or treatment methods for circulatory diseases wherein the particular target cardiac disease is cardiac hypertrophy, cardiac failure or myocardial infarction.

The invention still further relates to angiotensin II production suppressors comprising chymase inhibitors and ACE inhibitors as effective ingredients, and to a method of suppressing angiotensin II production by administering a chymase inhibitor and ACE inhibitor.

### Brief Description of the Drawings

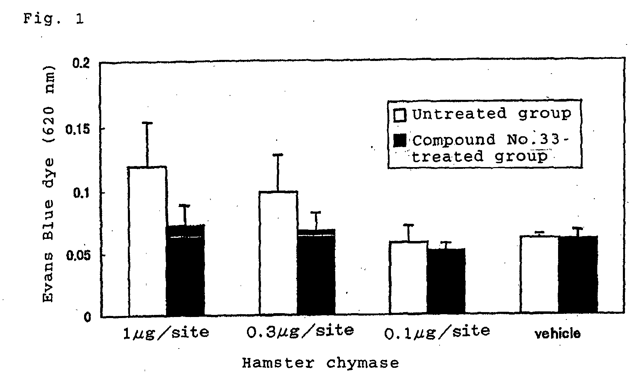

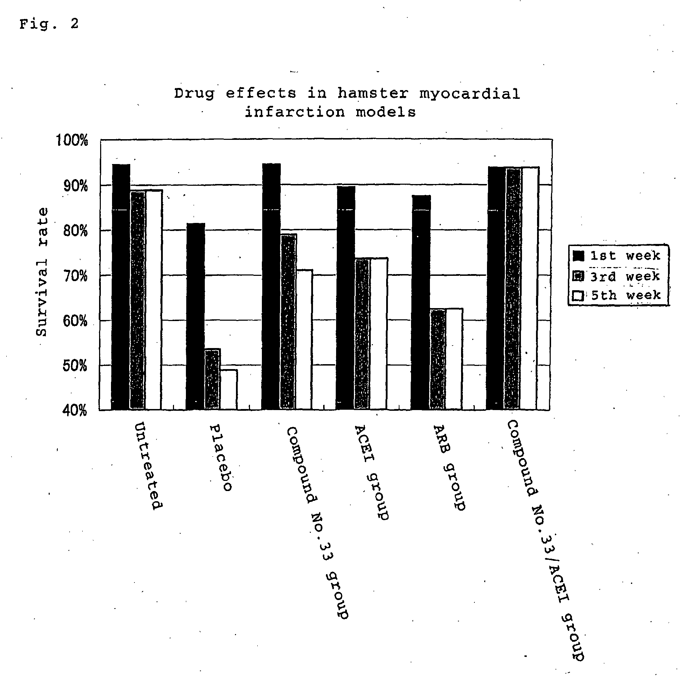

Fig. 1 is a bar graph showing the effect of a chymase inhibitor on accelerated vascular permeation. Fig. 2 is a bar graph showing the drug effect in a hamster myocardial infarction model.

### Best Mode for Carrying Out the Invention

The present invention relates to drugs comprising chymase inhibitors and ACE inhibitors as effective ingredients, to treatment agents for circulatory diseases, to angiotensin II production suppressors, to treatment methods involving administration of chymase inhibitors and ACE inhibitors, to treatment methods for circulatory diseases and to methods for suppressing production of angiotensin II.

According to the invention, a chymase inhibitor is used as a first effective ingredient, and an ACE inhibitor is used as a second effective ingredient.

ACE inhibitors are drugs whose utility for treatment of circulatory conditions such as hypertension has already been confirmed. Chymase inhibitors are effective for improving prognosis of myocardial infarction based on suppression of tissue local angiotensin II production, and this has been confirmed for the present invention. Thus, chymase inhibitors alone are also effective as drugs for circulatory diseases and suppressors of angiotensin II production. The invention employs a combination of an ACE inhibitor and a chymase inhibitor to thoroughly suppress angiotensin II production in local tissue, maintain the ACE inhibitor action of suppressing bradykinin decomposition and suppress various kinds of chymase-mediated physiological activity, for an overall superior effect compared to using the ACE inhibitor or the chymase inhibitor alone. This exhibited synergistic therapeutic effect is efficacious for treatment of circulatory diseases or suppression of angiotensin II production.

The drugs of the invention are chymase inhibitors and ACE inhibitors which are administered either simultaneously or separately at different times.

A drug of the invention may be in any form so long as it comprises a chymase inhibitor and an ACE inhibitor as effective ingredients. The form of a drug according to the invention may be, for example, a mixture composed mainly of the chymase inhibitor and ACE inhibitor, or else the chymase inhibitor and ACE inhibitor may be single agents in the form of independent agents not in admixture, with no particular restrictions so long as the chymase inhibitor and ACE inhibitor are in combination. Here, "mixture" refers to a combination of two or more effective ingredients in a single formulation, while "single agent" is one comprising a single specific effective ingredient in a single formulation.

A compounded form of a chymase inhibitor and an ACE inhibitor may be prepared, for example, by combining the chymase inhibitor ingredient and the ACE inhibitor ingredient in amounts sufficient to exhibit their respective drug effects, and formulating them in a dosage form such as tablets, capsules, a liquid drug, or the like. The timing for admixture of the chymase inhibitor and ACE inhibitor may be at the stage of preparing the mixed dosage form, or immediately before administration. When the admixture is carried out at the preparation stage, for example, the chymase inhibitor and ACE inhibitor components are compounded in their respective appropriate amounts and molded or packed. For molding, the agents may be either mixed or laminated in layers, with no particular restrictions. For preparation of a compounded mixture immediately prior to administration, for example, the chymase inhibitor and ACE inhibitor may be kept in independent states as liquid agents up until administration and the liquid agents combined at the time of administration, one agent in solid form such as tablets, pills, granules, powder or capsules may be dissolved in the other liquid agent, or both agents may be in solid form such as granules or powder and combined together. Admixture immediately prior to administration may be accomplished by hand, or there may be used a package allowing the two agents to be mixed together in a simple manner by cutting, drawing, tearing or pulling off. A compounded mixture may be in a dosage form such as tablets, pills, granules, powder, a liquid, a suspension, syrup or capsules.

A drug comprising the chymase inhibitor and ACE inhibitor each as independent single agents is a drug which permits the combined use of single agents of the chymase inhibitor and ACE inhibitor that can each be used alone. The respective forms of the agents may be either or both solid or liquid, with no particular restrictions.

A kit comprising the chymase inhibitor and ACE inhibitor of the invention is an assemblage wherein the chymase inhibitor and ACE inhibitor are arranged in a single system to facilitate their preparation. The assemblage of such a kit is not particularly restricted, and for example, the agents may be packaged in the same PTP or blister pack at the final stage of production, or they may be placed in the same purse upon prescription at the hospital or pharmacy.

A prophylactic or treatment method of the invention may involve administration of both the chymase inhibitor and ACE inhibitor agents, with no particular restrictions on the method of administration. The method of the invention may comprise simultaneous or separate administration of the chymase inhibitor and ACE inhibitor.

Examples of methods of administering the chymase inhibitor and ACE inhibitor include a method of administering a mixture of the chymase inhibitor and ACE inhibitor, a method of administering the chymase inhibitor and ACE inhibitor prepared as independent single agents, or a method of administering a compounded form of the chymase inhibitor and ACE inhibitor.

The chymase inhibitors and ACE inhibitors of the invention may be administered simultaneously or at separate starting times. When administered at separate starting times, they may be administered alternately, or one agent administered continuously and the other agent administered thereafter. They may also be administered the same number of times or a different number of times.

The administration method may be oral or parenteral for both agents, or one may be oral and the other parenteral.

Of the chymase inhibitors of the invention, the benzimidazole derivatives represented by formula (I) are preferably molded as a pharmaceutical composition into any of various dosage forms together with a pharmaceutically acceptable carrier and administered orally or parenterally, separately from the pharmaceutical composition containing the ACE inhibitor. As an alternative and preferred mode, a mixture or compounded form may be prepared with an appropriate ACE inhibitor for oral or parenteral administration.

The method and dosage for administration of the ACE inhibitor used in combination with the chymase inhibitor according to the invention will differ depending on the type of condition, the route of administration, and the symptoms, age, gender and body weight of the patient, and several different methods may be applied. For example, a mixture of the ACE inhibitor and chymase inhibitor may be prepared for simultaneous internal administration. When temocapril hydrochloride is used as the ACE inhibitor, for example, a mixture of 1-4 mg of temocapril hydrochloride and 1-10 mg of the chymase inhibitor may be prepared for oral internal use. Alternatively, the ACE inhibitor and chymase inhibitor may be prepared as separate tablets for individual administration. They are preferably taken simultaneously, but the dosages of each of the agents may be adjusted independently to best adapt to the symptoms and severity of the condition.

As examples of dosage forms of the pharmaceutical composition of the invention there may be mentioned, in the case of oral administration, tablets, pills, granules, powders, liquids, suspensions, syrups and capsules.

The method of molding tablets may be a common method using a pharmaceutically acceptable carrier such as an excipient, binder and/or disintegrator. Pills, granules and powders may also be molded by common methods using excipients and the like, as for tablets. The preparation method for a liquid, suspension or syrup may be a common method using a glycerin ester, alcohol, water and/or vegetable oil. A preparation method for capsules may entail filling granules, powder or a liquid into capsules of gelatin or the like.

A chymase inhibitor used according to the invention is preferably a benzimidazole derivative represented by formula (I) below as disclosed in the aforementioned patent specifications WO01/53291, WO01/53272 and WO00/03997, or a pharmaceutically acceptable salt thereof. 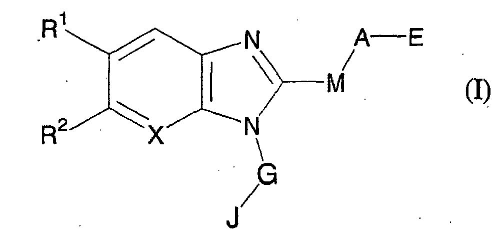 [wherein R¹ and R² may be the same or different and each independently represents a hydrogen atom, a halogen atom, a trihalomethyl group, cyano, hydroxyl, C₁₋₄ alkyl or C₁₋₄ alkoxy, or R¹ and R² may together form -O-CH₂-O-, -O-CH₂CH₂-O-, or -CH₂CH₂CH₂- (where in the case of -O-CH₂-O-, -O-CH₂CH₂-O- or -CH₂CH₂CH₂-, the carbon atoms may be substituted with one or more C₁₋₄ alkyl groups);
A represents a substituted or unsubstituted C₁₋₇ linear, cyclic or branched alkylene or alkenylene group, and may contain therein one or more from among -O-, -S-, -SO₂- and -NR³- (where R³ represents a hydrogen atom or a linear or branched C₁₋₆ alkyl group). Substituents for these groups include halogen atoms, hydroxyl, nitro, cyano, linear or branched C₁₋₆ alkyl, linear or branched C₁₋₆ alkoxy (including 2 or more adjacent ones forming an acetal bond), linear or branched C₁₋₆ alkylthio, linear or branched C₁₋₆ alkylsulfonyl, linear or branched C₁₋₆ acyl, linear or branched C₁₋₆ acylamino, trihalomethyl, trihalomethoxy, phenyl, oxo and phenoxy substituted with one or more halogen atoms. One or more of these substituents may independently substitute at any desired position on the alkylene or alkenylene group;
E represents -COOR³, -SO₃R³, -CONHR³, -SO₂NHR³, tetrazol-5-yl, 5-oxo-1,2,4-oxadiazol-3-yl or 5-oxo-1,2,4-thiadiazol-3-yl (where R³ is the same as defined above);
G represents a substituted or unsubstituted C₁₋₆ linear or branched alkylene group, and may contain therein one or more from among -O-, -S-, -SO₂- and -NR³- (where R³ is the same as defined above, and when such atoms or atomic groups are present they are not bonded directly to the benzimidazole ring). Substituents for these alkylene groups include halogen atoms, hydroxyl, nitro, cyano, linear or branched C₁₋₆ alkyl, linear or branched C₁₋₆ alkoxy (including 2 or more adjacent ones forming an acetal bond), trihalomethyl, trihalomethoxy, phenyl and oxo;
M represents a single bond or -S(O)ₘ- where m is an integer of 0-2;
J represents a substituted or unsubstituted C₄₋₁₀ heteroaryl group having on the ring one or more hetero atoms selected from the group consisting of oxygen, nitrogen and sulfur atoms, with the exception of pyridine. Substituents for these heteroaryl groups include halogen atoms, hydroxyl, nitro, cyano, linear or branched C₁₋₆ alkyl, linear or branched C₁₋₆ alkoxy (including 2 or more adjacent ones forming an acetal bond), linear or branched C₁₋₆ alkylthio, linear or branched C₁₋₆ alkylsulfonyl, linear or branched C₁₋₆ acyl, linear or branched C₁₋₆ acylamino, substituted or unsubstituted anilido, trihalomethyl, trihalomethoxy, phenyl, oxo, COOR³ (where R³ is the same as defined above), or phenoxy substituted with one or more halogen atoms. One or more of these substituents may independently substitute at any desired position on the ring; or J alternatively represents a substituted or unsubstituted C₁₋₆ linear, cyclic or branched alkyl or substituted or unsubstituted C₄₋₁₀ aryl group {Substituents for these groups include halogen atoms, hydroxyl, nitro, cyano, -COOR⁴ (where R⁴ represents a hydrogen atom or C₁₋₄ alkyl group), linear, cyclic or branched C₁₋₆ alkylene, C₁₋₆ linear or branched alkoxy (including 2 or more adjacent ones forming an acetal bond), C₁₋₆ linear or branched alkylthio, C₁₋₆ linear or branched alkylsulfonyl, C₁₋₆ linear or branched alkylsulfinyl, C₁₋₆ acyl, linear or branched C₁₋₆ acylamino, trihalomethyl, trihalomethoxy, phenyl, oxo or phenoxy substituted with one or more halogen atoms. One or more of these substituents may independently substitute at any desired position on the alkylene or aryl group. These substituents may also be in turn substituted with halogen atoms, hydroxyl, nitro, cyano, acyl, trihalomethyl, phenyl, oxo or optionally halogen-substituted phenoxy};
and X represents methine (-CH=) or a nitrogen atom.]

The following are preferred compounds represented by formula (I).

R¹ and R² may be the same or different and each independently represents a hydrogen atom, a halogen atom, a trihalomethyl group, cyano, hydroxyl, C₁₋₄ alkyl or C₁₋₄ alkoxy, or R¹ and R² may together form -O-CH₂-O-, -O-CH₂CH₂-O-, or -CH₂CH₂CH₂-, where the carbon atoms may be substituted with one or more C₁₋₄ alkyl groups.

As C₁₋₄ alkyl groups for R¹ and R² there may be mentioned specifically methyl, ethyl, (n-, i-)propyl and (n-, i-, s-, t-)butyl, and preferably methyl. As C₁₋₄ alkoxy groups there may be mentioned specifically methoxy, ethoxy, (n-, i-)propyloxy and (n-, i-, s-, t-)butyloxy.

As preferred groups for R¹ and R² there may be mentioned hydrogen, halogen atoms, trihalomethyl, cyano, hydroxyl, C₁₋₄ alkyl or C₁₋₄ alkoxy, among which hydrogen, halogen atoms, trihalomethyl, cyano, C₁₋₄ alkyl and C₁₋₄ alkoxy are preferred, hydrogen, chlorine, fluorine, trifluoromethyl, methyl, methoxy and ethoxy are more preferred, and hydrogen, methyl, methoxy and ethoxy are especially preferred.

A represents a substituted or unsubstituted C₁₋₇ linear, cyclic or branched alkylene or alkenylene group. As unsubstituted C₁₋₇ linear, cyclic or branched alkylene groups there may be mentioned methylene, ethylene, (n-, i-)propylene, 2,2-dimethylpropylene, (n-, i-, t-)butylene, 1,1-dimethylbutylene, n-pentylene or cyclohexylene, preferably ethylene, n-propylene, 2,2-dimethylpropylene or (n-, t-)butylene, more preferably n-propylene or 2,2-dimethylpropylene, and most preferably n-propylene. As unsubstituted C₁₋₇ linear or branched alkenylene groups there may be mentioned vinylene, propenylene, butenylene or pentenylene. These alkylene or alkenylene groups may also contain one or more from among -O-, -S-, -SO₂- and -NR³- (where R³ represents a hydrogen atom or a linear or branched C₁₋₆ alkyl group), with the proviso that such atoms or atomic groups are not bonded directly to M. Specifically there may be mentioned groups at positions between ethylene, n-propylene or (n-, t-)butylene. More specifically, there may be mentioned -CH₂OCH₂-, -CH₂OCH₂CH₂-, -CH₂SCH₂-, -CH₂SCH₂CH₂-, -CH₂SO₂CH₂-, -CH₂SO₂CH₂CH₂-, -CH₂NR⁴CH₂- or -CH₂NR⁴CH₂CH₂-, and preferably -CH₂OCH₂-, -CH₂SCH₂- or -CH₂SO₂CH₂-.

Substituents for these alkylene or alkenylene groups include halogen atoms, hydroxyl, nitro, cyano, linear or branched C₁₋₆ alkyl, linear or branched C₁₋₆ alkoxy (including 2 or more adjacent ones forming an acetal bond), linear or branched C₁₋₆ alkylthio, linear or branched C₁₋₆ alkylsulfonyl, linear or branched C₁₋₆ acyl, linear or branched C₁₋₆ acylamino, trihalomethyl, trihalomethoxy, phenyl, oxo or phenoxy substituted with one or more halogen atoms. One or more of these substituents may independently substitute at any desired position on the alkylene or alkenylene.

As halogen atoms for the substituent of A there may be mentioned fluorine, chlorine, bromine or iodine, and preferably fluorine or chlorine.

As linear or branched C₁₋₆ alkyl groups for the substituent of A there may be mentioned specifically methyl, ethyl, (n-, i-)propyl or (n-, i-, s-, t-)butyl, preferably methyl or ethyl, and more preferably methyl.

As linear or branched C₁₋₆ alkoxy groups for the substituent of A there may be mentioned specifically methoxy, ethoxy, (n-, i-)propyloxy or (n-, i-, s-, t-)butyloxy, preferably methoxy or ethoxy, and more preferably methoxy.

As linear or branched C₁₋₆ alkylthio groups for the substituent of A there may be mentioned specifically methylthio, ethylthio, (n-, i-)propylthio or (n-, i-, s-, t-)butylthio, preferably methylthio or ethylthio, and more preferably methylthio.

As linear or branched C₁₋₆ alkylsulfonyl groups for the substituent of A there may be mentioned specifically methylsulfonyl, ethylsulfonyl, (n-, i-)propylsulfonyl or (n-, i-, s-, t-)butylsulfonyl, preferably methylsulfonyl or ethylsulfonyl, and more preferably methylsulfonyl.

As linear or branched C₁₋₆ acyl groups for the substituent of A there may be mentioned acetyl, ethylcarbonyl, (n-, i-)propylcarbonyl or (n-, i-, s-, t-)carbonyl, preferably acetyl or ethylcarbonyl, and more preferably acetyl.

As linear or branched C₁₋₆ acylamino groups for the substituent of A there may be mentioned specifically acetylamino, ethylcarbonylamino, (n-, i-)propylcarbonylamino or (n-, i-, s-, t-)carbonylamino, preferably acetylamino or ethylcarbonylamino, and more preferably acetylamino.

As trihalomethyl groups for the substituent of A there may be mentioned specifically trifluoromethyl, tribromomethyl or trichloromethyl, and preferably trifluoromethyl.

Preferred as A are substituted or unsubstituted C₁₋₇ linear, cyclic or branched alkylene groups {which may contain therein one or more from among -O-, -S-, -SO₂- and -NR³- (where R³ is the same as defined above), with the proviso that such atoms or atomic groups are not bonded directly to M}. There may be mentioned preferably -CH₂CH₂-, -CH₂CH₂CH₂-, -CH₂C(=O)CH₂-, -CH₂OCH₂-, -CH₂SCH₂-, -CH₂S(=O)CH₂-, -CH₂CF₂CH₂-, -CH₂SO₂CH₂-, -CH₂CH₂CH₂CH₂-, -CH₂C(CH₃)₂CH₂-, -CH₂SO₂CH₂CH₂-, -CH₂C(=O)CH₂CH₂-, -CH₂C(=O)(CH₃)₂CH₂- or -CH₂C(=O)C(=O)CH₂-, more preferably -CH₂CH₂-, -CH₂CH₂CH₂-, -CH₂C(=O)CH₂-, -CH₂OCH₂-, -CH₂SCH₂-, -CH₂S(=O)CH₂-, -CH₂CF₂CH₂-, -CH₂SO₂CH₂- or -CH₂C(CH₃)₂CH₂-, even more preferably -CH₂CH₂-, -CH₂CH₂CH₂- or -CH₂C(CH₃)₂CH₂-, and most preferably -CH₂CH₂CH₂-.

E represents -COOR³, -SO₃R³, -CONHR³, -SO₂NHR³, tetrazol-5-yl, 5-oxo-1,2,4-oxadiazol-3-yl or 5-oxo-1,2,4-thiadiazol-3-yl (where R³ represents a hydrogen atom or a linear or branched C₁₋₆ alkyl group).

As R³ there may be mentioned specifically hydrogen, methyl, ethyl, (n-, i-)propyl or (n-, i-, s-, t-)butyl, preferably hydrogen, methyl or ethyl, and most preferably hydrogen.

As E there may be mentioned preferably -COOR³, -SO₃R³ or tetrazol-5-yl, more preferably -COOR³, and most preferably -COOH.

G represents a substituted or unsubstituted C₁₋₆ linear or branched alkylene group, and may contain therein one or more from among -O-, -S-, -SO₂- and -NR³-, where R³ is the same as defined above, with the proviso that when such hetero atoms or atomic groups are present they are not bonded directly to the benzimidazole ring. Substituents for these alkylene groups include halogen atoms, hydroxyl, nitro, cyano, linear or branched C₁₋₆ alkyl, linear or branched C₁₋₆ alkoxy (including 2 or more adjacent ones forming an acetal bond), trihalomethyl, trihalomethoxy, phenyl or oxo. There may be mentioned as specific examples -CH₂-, -CH₂CH₂-, -CH₂CO-, -CH₂CH₂O-, -CH₂CONH-, -CO-, -SO₂-, -CH₂SO₂-, -CH₂S- or -CH₂CH₂S-, preferably -CH₂-, -CH₂CH₂-, -CH₂CO- or -CH₂CH₂O-, more preferably -CH₂- or -CH₂CH₂-, and especially -CH₂-, with the proviso that these groups are bonded at the left to the benzimidazole 1-position (N atom) and at the right to J.

M represents a single bond or -S(O)ₘ- where m is an integer of 0-2. There may be mentioned preferably -S- or -SO₂-, and most preferably -S-.

J is represented by J¹ or J².

J¹ represents a substituted or unsubstituted C₄₋₁₀ heteroaryl group having on the ring one or more hetero atoms selected from the group consisting of oxygen, nitrogen and sulfur atoms, with the exception of pyridine. J² represents a substituted or unsubstituted C₁₋₆ linear, cyclic or branched alkyl group or a substituted or unsubstituted C₄₋₁₀ aryl group. These are limited to chemically synthesizable compounds.

As unsubstituted C₄₋₁₀ heteroaryl groups having on the ring one or more hetero atoms selected from the group consisting of oxygen, nitrogen and sulfur atoms there may be mentioned specifically furyl, thienyl, thiazolyl, pyrimidinyl, oxazolyl, isooxazolyl, benzofuryl, benzimidazolyl, quinolyl, isoquinolyl, quinoxalinyl, benzoxadiazolyl, benzothiadiazolyl, indolyl, benzothiazolyl, benzothienyl or benzoisoxazolyl, preferably bicyclic heteroaromatic rings, more preferably benzofuryl, benzimidazolyl, quinolyl, isoquinolyl, quinoxalinyl, benzoxadiazolyl, benzothiadiazolyl, indolyl, benzothiazolyl, benzothienyl or benzoisoxazolyl, and most preferably benzothienyl or indolyl.

Substituents for these heteroaryl or aryl groups include halogen atoms, hydroxyl, nitro, cyano, linear or branched C₁₋₆ alkyl, linear or branched C₁₋₆ alkoxy (including 2 or more adjacent ones forming an acetal bond), linear or branched C₁₋₆ alkylthio, linear or branched C₁₋₆ alkylsulfonyl, linear or branched C₁₋₆ acyl, linear or branched C₁₋₆ acylamino, substituted or unsubstituted anilido, trihalomethyl, trihalomethoxy, phenyl or phenoxy substituted with one or more halogen atoms. One or more of these substituents may independently substitute at any desired position on the ring.

As halogen atoms for the substituent of J¹ there may be mentioned fluorine, chlorine, bromine or iodine, and preferably fluorine or chlorine.

As linear or branched C₁₋₆ alkyl groups for the substituent of J¹ there may be mentioned specifically methyl, ethyl, (n-, i-)propyl or (n-, i-, s-, t-)butyl, preferably methyl or ethyl, and more preferably methyl.

As linear or branched C₁₋₆ alkoxy groups for the substituent of J¹ there may be mentioned specifically methoxy, ethoxy, (n-, i-)propyloxy, (n-, i-, s-, t-)butyloxy or methylenedioxy, preferably methoxy or ethoxy, and more preferably methoxy.

As linear or branched C₁₋₆ alkylthio groups for the substituent of J¹ there may be mentioned specifically methylthio, ethylthio, (n-, i-)propylthio or (n-, i-, s-, t-)butylthio, preferably methylthio or ethylthio, and more preferably methylthio.

As linear or branched C₁₋₆ alkylsulfonyl groups for the substituent of J¹ there may be mentioned specifically methylsulfonyl, ethylsulfonyl, (n-, i-)propylsulfonyl or (n-, i-, s-, t-)butylsulfonyl, preferably methylsulfonyl or ethylsulfonyl, and more preferably methylsulfonyl.

As linear or branched C₁₋₆ acyl groups for the substituent of J¹ there may be mentioned acetyl, ethylcarbonyl, (n-, i-)propylcarbonyl or (n-, i-, s-, t-)carbonyl, preferably acetyl or ethylcarbonyl, and more preferably acetyl.

As linear or branched C₁₋₆ acylamino groups for the substituent of J¹ there may be mentioned specifically acetylamino, ethylcarbonylamino, (n-, i-)propylcarbonylamino or (n-, i-, s-, t-)carbonylamino, preferably acetylamino or ethylcarbonylamino, and more preferably acetylamino.

As trihalomethyl groups for the substituent of J¹ there may be mentioned specifically trifluoromethyl, tribromomethyl or trichloromethyl.

J² represents a substituted or unsubstituted C₁₋₆ linear, cyclic or branched alkyl group or a substituted or unsubstituted C₄₋₁₀ aryl group. As unsubstituted C₁₋₆ linear, cyclic or branched alkyl groups there may be mentioned methyl, ethyl, (n-, i-)propyl, (n-, i-, s-, t-)butyl, cyclopentyl or cyclohexyl.

As substituents for J² there may be mentioned halogen atoms, hydroxyl, nitro, cyano, -COOR⁴ (where R⁴ represents a hydrogen atom or a C₁₋₄ alkyl group), linear, cyclic or branched C₁₋₆ alkylene, C₁₋₆ linear or branched alkoxy (in which case an acetal bond may be formed by substituents at adjacent positions), C₁₋₆ linear or branched alkylthio, C₁₋₆ linear or branched alkylsulfonyl, C₁₋₆ linear or branched alkylsulfinyl, linear or branched C₁₋₆ acyl, linear or branched C₁₋₆ acylamino, trihalomethyl, trihalomethoxy, phenyl, oxo or phenoxy substituted with one or more halogen atoms. One or more of these substituents may independently substitute at any desired position on the alkyl or aryl group. These substituents may also be in turn substituted with halogen atoms, hydroxyl, nitro, cyano, acyl, trihalomethyl, phenyl, oxo or optionally halogen-substituted phenoxy.

Preferred among the above for J² are substituted or unsubstituted C₄₋₁₀ aryl groups. Specifically preferred are groups represented by the following formulas (XII) and (XIII): 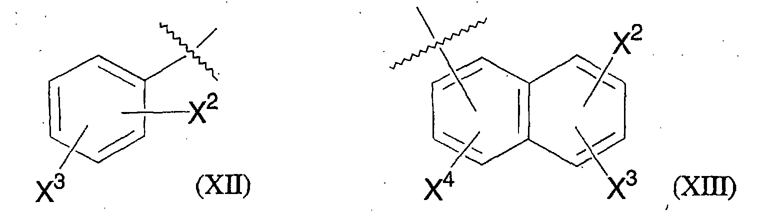 [wherein X², X³ and X⁴ may be the same or different and each independently represents a hydrogen atom, halogen atom, hydroxyl, nitro, cyano, trihalomethyl, trihalomethoxy, COOR⁴ (where R4 represents a hydrogen atom or a C₁₋₄ alkyl group), substituted or unsubstituted C₁₋₃ linear or branched alkyl, substituted or unsubstituted C₁₋₃ linear or branched alkoxy, substituted or unsubstituted C₁₋₃ linear or branched alkylthio, substituted or unsubstituted C₁₋₃ linear or branched alkylsulfonyl or substituted or unsubstituted C₁₋₃ linear or branched alkylsulfinyl. There are no restrictions on the substitution positions of X², X³ and X⁴ on the benzene ring or naphthalene ring.]

As halogen atoms for the substituent of J² there may be mentioned fluorine, chlorine, bromine or iodine, and preferably fluorine or chlorine. Trifluoromethyl is preferred as a trihalomethyl group, and trifluoromethoxy is preferred as a trihalomethoxy group. As unsubstituted C₁₋₃ linear or branched alkyl groups there may be mentioned specifically methyl, ethyl or (n-, i-)propyl. As unsubstituted C₁₋₃ linear or branched alkoxy groups there may be mentioned specifically methoxy, ethoxy or (n-, i-)propyloxy. As unsubstituted C₁₋₃ linear or branched alkylthio groups there may be mentioned specifically methylthio, ethylthio or (n-, i-)propylthio. As unsubstituted C₁₋₃ linear or branched alkylsulfonyl groups there may be mentioned specifically methylsulfonyl, ethylsulfonyl or (n-, i-)propylsulfonyl. As unsubstituted C₁₋₃ linear or branched alkylsulfinyl groups there may be mentioned specifically methylsulfinyl, ethylsulfinyl or (n-, i-)propylsulfinyl.

These substituents for J² may also be in turn substituted with halogen atoms, hydroxyl, nitro, cyano, acyl, trihalomethyl, phenyl, oxo or optionally halogen-substituted phenoxy.

There are no particular restrictions on the substituent positions for X², X³ and X⁴ in formulas (XII) and (XIII), but a combination of positions 2- and 3- or a combination of positions 2- and 5- is preferred in formula (XII). For formula (XIII), combinations of positions 4-, 7-, 8-, positions 4-, 6-, 8- and positions 6-, 7-, 8- are preferred.

As preferred compounds for J² there may be mentioned 2-methylphenyl, 2-ethylphenyl, 3-trifluoromethylphenyl, 2-ethoxyphenyl, 3-methoxyphenyl, 2-chlorophenyl, 2-trifluorophenyl, 2,3-methylenedioxyphenyl, 2-methyl-3-methoxyphenyl, 2-trifluoromethyl-3-methoxyphenyl, 2-methyl-3-trifluoromethoxyphenyl, 2,3-dimethylphenyl, 2,3-dichlorophenyl, 2,3-dimethoxyphenyl, 2,5-dimethoxyphenyl, 2,5-dimethylphenyl, 2,5-dichlorophenyl, 2,5-ditrifluoromethylphenyl, 1-naphthyl, 2-naphthyl, 8-methyl-1-naphthyl, 7-methyl-1-naphthyl, 6,8-dimethyl-2-naphthyl and 4,6,8-trimethyl-1-naphthyl.

X in formula (I) represents -CH= or a nitrogen atom, and preferably -CH=.

The preferred compounds represented by formula (I) include various compound families having the combinations of groups mentioned above as being preferred, The compounds listed in Table 1 below are preferred but not restrictive. Particularly preferred compounds among those listed in Table 1 are Compound Nos. 2, 6, 7, 9, 10, 20, 22, 24, 26, 27, 31, 33, 43, 45, 56, 60, 62, 92, 128, 164, 182, 187, 189, 201, 202, 204, 206, 240 and 242.

A1 and J1-J36 in Table 1 are the groups shown as the following formulas. Groups in J1-J36 indicated by "-" with no symbol are all "-CH₃".

E, G, M, m and X in the formulas have the same definitions as given above, and while compounds where E is COOH, G is CH₂, M is S (m=0) or a single bond (indicated as "-" in the table) and X is -CH= are listed as representative compounds, there is no intended restriction to these.

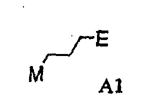

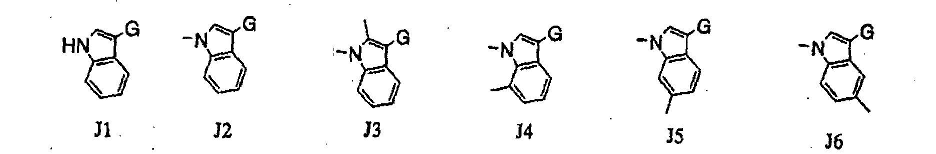

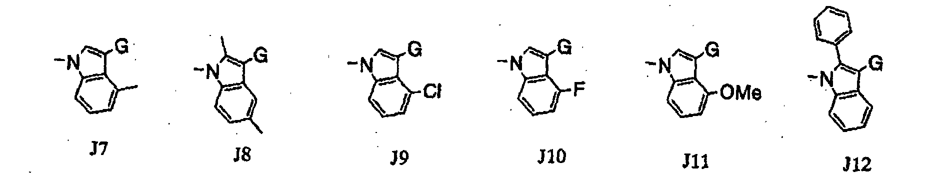

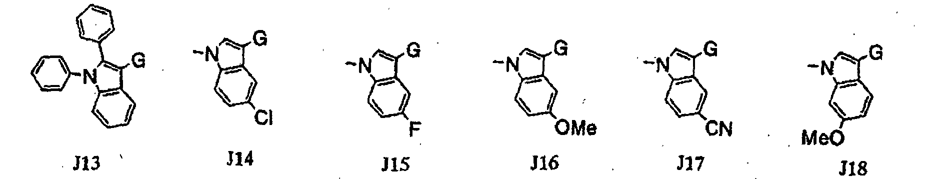

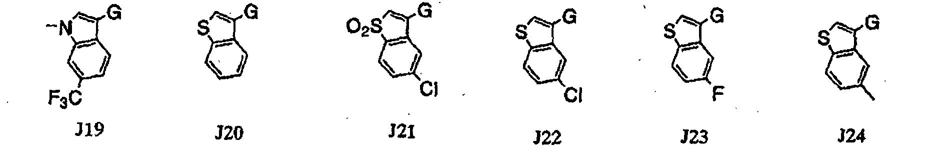

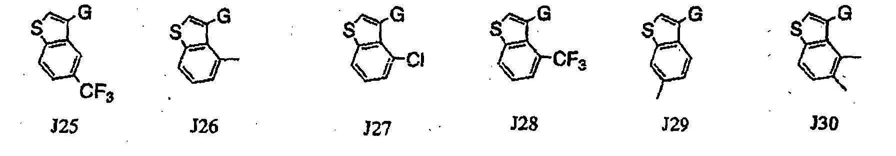

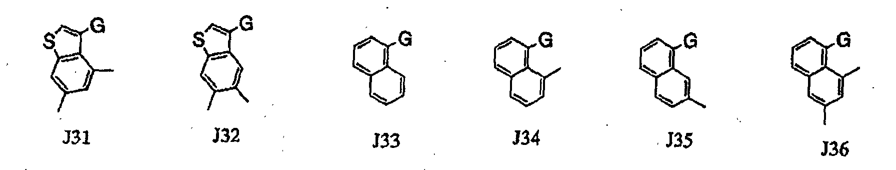

**Table 1**

| Compound No. | R1 | R2 | A | J | M |
|---|---|---|---|---|---|
| 1 | H | H | A1 | J1 | S |
| 2 | H | H | A1 | J2 | S |
| 3 | H | H | A1 | J3 | S |
| 4 | H | H | A1 | J4 | S |
| 5 | H | H | A1 | J5 | S |
| 6 | H | H | A1 | J6 | S |
| 7 | H | H | A1 | J7 | S |
| 8 | H | H | A1 | J8 | S |
| 9 | H | H | A1 | J9 | S |
| 10 | H | H | A1 | J10 | S |
| 11 | H | H | A1 | J11 | S |
| 12 | H | H | A1 | J12 | S |
| 13 | H | H | A1 | J13 | S |
| 14 | H | H | A1 | J14 | S |
| 15 | H | H | A1 | J15 | S |
| 16 | H | H | A1 | J16 | S |
| 17 | H | H | A1 | J17 | S |
| 18 | H | H | A1 | J18 | S |
| 19 | H | H | A1 | J19 | S |
| 20 | H | H | A1 | J20 | S |
| 21 | H | H | A1 | J21 | S |
| 22 | H | H | A1 | J22 | S |
| 23 | H | H | A1 | J23 | S |
| 24 | H | H | A1 | J24 | S |
| 25 | H | H | A1 | J25 | S |
| 26 | H | H | A1 | J26 | S |
| 27 | H | H | A1 | J27 | S |
| 28 | H | H | A1 | J28 | S |
| 29 | H | H | A1 | J29 | S |
| 30 | H | H | A1 | J30 | S |
| 31 | H | H | A1 | J31 | S |
| 32 | H | H | A1 | J32 | S |
| 33 | H | H | A1 | J33 | S |
| 34 | H | H | A1 | J34 | S |
| 35 | H | H | A1 | J35 | S |
| 36 | H | H | A1 | J36 | S |
| 37 | MeO | H | A1 | J1 | S |
| 38 | MeO | H | A1 | J2 | S |
| 39 | MeO | H | A1 | J3 | S |
| 40 | MeO | H | A1 | J4 | S |
| 41 | MeO | H | A1 | J5 | S |
| 42 | MeO | H | A1 | J6 | S |
| 43 | MeO | H | A1 | J7 | S |
| 44 | MeO | H | A1 | J8 | S |
| 45 | MeO | H | A1 | J9 | S |
| 46 | MeO | H | A1 | J10 | S |
| 47 | MeO | H | A1 | J11 | S |
| 48 | MeO | H | A1 | J12 | S |
| 49 | MeO | H | A1 | J13 | S |
| 50 | MeO | H | A1 | J14 | S |
| 51 | MeO | H | A1 | J15 | S |
| 52 | MeO | H | A1 | J16 | S |
| 53 | MeO | H | A1 | J17 | S |
| 54 | MeO | H | A1 | J18 | S |
| 55 | MeO | H | A1 | J19 | S |
| 56 | MeO | H | A1 | J20 | S |
| 57 | MeO | H | A1 | J21 | S |
| 58 | MeO | H | A1 | J22 | S |
| 59 | MeO | H | A1 | J23 | S |
| 60 | MeO | H | A1 | J24 | S |
| 61 | MeO | H | A1 | J25 | S |
| 62 | MeO | H | A1 | J26 | S |
| 63 | MeO | H | A1 | J27 | S |
| 64 | MeO | H | A1 | J28 | S |
| 65 | MeO | H | A1 | J29 | S |
| 66 | MeO | H | A1 | J30 | S |
| 67 | MeO | H | A1 | J31 | S |
| 68 | MeO | H | A1 | J32 | S |
| 69 | MeO | H | A1 | J33 | S |
| 70 | MeO | H | A1 | J34 | S |
| 71 | MeO | H | A1 | J35 | S |
| 72 | MeO | H | A1 | J36 | S |
| 73 | CN | H | A1 | J1 | S |
| 74 | CN | H | A1 | J2 | S |
| 75 | CN | H | A1 | J3 | S |
| 76 | CN | H | A1 | J4 | S |
| 77 | CN | H | A1 | J5 | S |
| 78 | CN | H | A1 | J6 | S |
| 79 | CN | H | A1 | J7 | S |
| 80 | CN | H | A1 | J8 | S |
| 81 | CN | H | A1 | J9 | S |
| 82 | CN | H | A1 | J10 | S |
| 83 | CN | H | A1 | J11 | S |
| 84 | CN | H | A1 | J12 | S |
| 85 | CN | H | A1 | J13 | S |
| 86 | CN | H | A1 | J14 | S |
| 87 | CN | H | A1 | J15 | S |
| 88 | CN | H | A1 | J16 | S |
| 89 | CN | H | A1 | J17 | S |
| 90 | CN | H | A1 | J18 | S |
| 91 | CN | H | A1 | J19 | S |
| 92 | CN | H | A1 | J20 | S |
| 93 | CN | H | A1 | J21 | S |
| 94 | CN | H | A1 | J22 | S |
| 95 | CN | H | A1 | J23 | S |
| 96 | CN | H | A1 | J24 | S |
| 97 | CN | H | A1 | J25 | S |
| 98 | CN | H | A1 | J26 | S |
| 99 | CN | H | A1 | J27 | S |
| 100 | CN | H | A1 | J28 | S |
| 101 | CN | H | A1 | J29 | S |
| 102 | CN | H | A1 | J30 | S |
| 103 | CN | H | A1 | J31 | S |
| 104 | CN | H | A1 | J32 | S |
| 105 | CN | H | A1 | J33 | S |
| 106 | CN | H | A1 | J34 | S |
| 107 | CN | H | A1 | J35 | S |
| 108 | CN | H | A1 | J36 | S |
| 109 | Me | H | A1 | J1 | S |
| 110 | Me | H | A1 | J2 | S |
| 111 | Me | H | A1 | J3 | S |
| 112 | Me | H | A1 | J4 | S |
| 113 | Me | H | A1 | J5 | S |
| 114 | Me | H | A1 | J6 | S |
| 115 | Me | H | A1 | J7 | S |
| 116 | Me | H | A1 | J8 | S |
| 117 | Me | H | A1 | J9 | S |
| 118 | Me | H | A1 | J10 | S |
| 119 | Me | H | A1 | J11 | S |
| 120 | Me | H | A1 | J12 | S |
| 121 | Me | H | A1 | J13 | S |
| 122 | Me | H | A1 | J14 | S |
| 123 | Me | H | A1 | J15 | S |
| 124 | Me | H | A1 | J16 | S |
| 125 | Me | H | A1 | J17 | S |
| 126 | Me | H | A1 | J18 | S |
| 127 | Me | H | A1 | J19 | S |
| 128 | Me | H | A1 | J20 | S |
| 129 | Me | H | A1 | J21 | S |
| 130 | Me | H | A1 | J22 | S |
| 131 | Me | H | A1 | J23 | S |
| 132 | Me | H | A1 | J24 | S |
| 133 | Me | H | A1 | J25 | S |
| 134 | Me | H | A1 | J26 | S |
| 135 | Me | H | A1 | J27 | S |
| 136 | Me | H | A1 | J28 | S |
| 137 | Me | H | A1 | J29 | S |
| 138 | Me | H | A1 | J30 | S |
| 139 | Me | H | A1 | J31 | S |
| 140 | Me | H | A1 | J32 | S |
| 141 | Me | H | A1 | J33 | S |
| 142 | Me | H | A1 | J34 | S |
| 143 | Me | H | A1 | J35 | S |
| 144 | Me | H | A1 | J36 | S |
| 145 | H | Me | A1 | J1 | S |
| 146 | H | Me | A1 | J2 | S |
| 147 | H | Me | A1 | J3 | S |
| 148 | H | Me | A1 | J4 | S |
| 149 | H | Me | A1 | J5 | S |
| 150 | H | Me | A1 | J6 | S |
| 151 | H | Me | A1 | J7 | S |
| 152 | H | Me | A1 | J8 | S |
| 153 | H | Me | A1 | J9 | S |
| 154 | H | Me | A1 | J10 | S |
| 155 | H | Me | A1 | J11 | S |
| 156 | H | Me | A1 | J12 | S |
| 157 | H | Me | A1 | J13 | S |
| 158 | H | Me | A1 | J14 | S |
| 159 | H | Me | A1 | J15 | S |
| 160 | H | Me | A1 | J16 | S |
| 161 | H | Me | A1 | J17 | S |
| 162 | H | Me | A1 | J18 | S |
| 163 | H | Me | A1 | J19 | S |
| 164 | H | Me | A1 | J20 | S |
| 165 | H | Me | A1 | J21 | S |
| 166 | H | Me | A1 | J22 | S |
| 167 | H | Me | A1 | J23 | S |
| 168 | H | Me | A1 | J24 | S |
| 169 | H | Me | A1 | J25 | S |
| 170 | H | Me | A1 | J26 | S |
| 171 | H | Me | A1 | J27 | S |
| 172 | H | Me | A1 | J28 | S |
| 173 | H | Me | A1 | J29 | S |
| 174 | H | Me | A1 | J30 | S |
| 175 | H | Me | A1 | J31 | S |
| 176 | H | Me | A1 | J32 | S |
| 177 | H | Me | A1 | J33 | S |
| 178 | H | Me | A1 | J34 | S |
| 179 | H | Me | A1 | J35 | S |
| 180 | H | Me | A1 | J36 | S |
| 181 | Me | Me | A1 | J1 | S |
| 182 | Me | Me | A1 | J2 | S |
| 183 | Me | Me | A1 | J3 | S |
| 184 | Me | Me | A1 | J4 | S |
| 185 | Me | Me | A1 | J5 | S |
| 186 | Me | Me | A1 | J6 | S |
| 187 | Me | Me | A1 | J7 | S |
| 188 | Me | Me | A1 | J8 | S |
| 189 | Me | Me | A1 | J9 | S |
| 190 | Me | Me | A1 | J10 | S |
| 191 | Me | Me | A1 | J11 | S |
| 192 | Me | Me | A1 | J12 | S |
| 193 | Me | Me | A1 | J13 | S |
| 194 | Me | Me | A1 | J14 | S |
| 195 | Me | Me | A1 | J15 | S |
| 196 | Me | Me | A1 | J16 | S |
| 197 | Me | Me | A1 | J17 | S |
| 198 | Me | Me | A1 | J18 | S |
| 199 | Me | Me | A1 | J19 | S |
| 200 | Me | Me | A1 | J20 | S |
| 201 | Me | Me | A1 | J21 | S |
| 202 | Me | Me | A1 | J22 | S |
| 203 | Me | Me | A1 | J23 | S |
| 204 | Me | Me | A1 | J24 | S |
| 205 | Me | Me | A1 | J25 | S |
| 206 | Me | Me | A1 | J26 | S |
| 207 | Me | Me | A1 | J27 | S |
| 208 | Me | Me | A1 | J28 | S |
| 209 | Me | Me | A1 | J29 | S |
| 210 | Me | Me | A1 | J30 | S |
| 211 | Me | Me | A1 | J31 | S |
| 212 | Me | Me | A1 | J32 | S |
| 213 | Me | Me | A1 | J33 | S |
| 214 | Me | Me | A1 | J34 | S |
| 215 | Me | Me | A1 | J35 | S |
| 216 | Me | Me | A1 | J36 | S |
| 217 | H | MeO | A1 | J1 | S |
| 218 | H | MeO | A1 | J2 | S |
| 219 | H | MeO | A1 | J3 | S |
| 220 | H | MeO | A1 | J4 | S |
| 221 | H | MeO | A1 | J5 | S |
| 222 | H | MeO | A1 | J6 | S |
| 223 | H | MeO | A1 | J7 | S |
| 224 | H | MeO | A1 | J8 | S |
| 225 | H | MeO | A1 | J9 | S |
| 226 | H | MeO | A1 | J10 | S |
| 227 | H | MeO | A1 | J11 | S |
| 228 | H | MeO | A1 | J12 | S |
| 229 | H | MeO | A1 | J13 | S |
| 230 | H | MeO | A1 | J14 | S |
| 231 | H | MeO | A1 | J15 | S |
| 232 | H | MeO | A1 | J16 | S |
| 233 | H | MeO | A1 | J17 | S |
| 234 | H | MeO | A1 | J18 | S |
| 235 | H | MeO | A1 | J19 | S |
| 236 | H | MeO | A1 | J20 | S |
| 237 | H | MeO | A1 | J21 | S |
| 238 | H | MeO | A1 | J22 | S |
| 239 | H | MeO | A1 | J23 | S |
| 240 | H | MeO | A1 | J24 | S |
| 241 | H | MeO | A1 | J25 | S |
| 242 | H | MeO | A1 | J26 | S |
| 243 | H | MeO | A1 | J27 | S |
| 244 | H | MeO | A1 | J28 | S |
| 245 | H | MeO | A1 | J29 | S |
| 246 | H | MeO | A1 | J30 | S |
| 247 | H | MeO | A1 | J31 | S |
| 248 | H | MeO | A1 | J32 | S |
| 249 | H | MeO | A1 | J33 | S |
| 250 | H | MeO | A1 | J34 | S |
| 251 | H | MeO | A1 | J35 | S |
| 252 | H | MeO | A1 | J36 | S |

As chymase inhibitors according to the invention there may be mentioned those represented by the following formula (II) as described in WO00/005204. 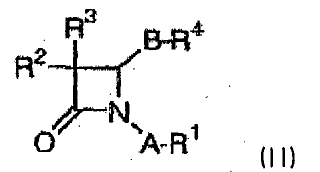 [wherein A is a single bond, -CO-, -COO-, -COCO-, -CONH- or -SO₂-, R¹ is optionally substituted lower alkyl, optionally substituted lower alkenyl, optionally substituted lower alkynyl, optionally substituted cycloalkyl, optionally substituted lower cycloalkenyl or optionally substituted aryl, (and R¹ may be hydrogen when A is a single bond, -CO-, -COCO-, -CONH- or -SO₂-), R² and R³ are each independently hydrogen, a halogen, optionally substituted lower alkyl, optionally substituted lower alkoxycarbonyl, optionally substituted acyl, optionally substituted amino, optionally substituted carbamoyl or optionally substituted aryl, B is a single bond, -S-, -O-, -S-S-, -SO- or -SO₂-, R⁴ is a hydrogen, optionally substituted lower alkyl, optionally substituted aryl, an optionally substituted heterocycle or, when B is a single bond, -S-, -O-, -SO- or -SO₂-, optionally substituted acyl.]

Of the chymase inhibitors represented by formula (II), 4-[1-{[bis(4-methylphenyl)methyl]carbamoyl}-3-(2-ethoxybenzyl)-4-oxoazetidin-2-yloyl]benzoic acid has already been reported to exhibit an effect when administered alone to hamster myocardial infarction models (Life Sci. 2002, Vol.71, p.437), and is therefore expected to have a notable effect when used in combination with ACE inhibitors for circulatory diseases.

As chymase inhibitors according to the invention there may also be mentioned the chymase inhibitors described in WO98/09949 which are represented by the following formula (III): 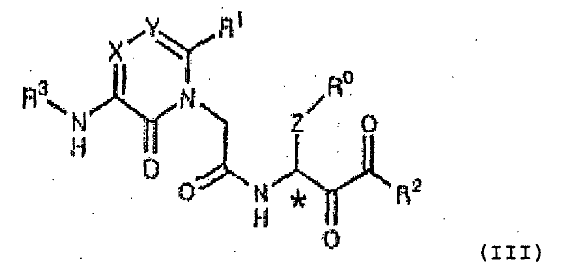 [wherein R⁰ is phenyl, optionally with one or more substituents on the ring selected from among those of Group A defined as follows. (Group A: a halogen, nitro, hydroxyl, lower alkoxy, lower alkyl or halogeno-substituted lower alkyl.) R¹ is (i) aryl, (ii) heteroaryl or (iii) C₁₋₆ linear, branched or cyclic alkyl, optionally each independently having one or more substituents defined according to Group A; R¹ may optionally have on the aforementioned groups (i) to (iii) one or more substituents selected from among those of Group B consisting of ORa, COORa, CONRbRc, NRbRc, NRbCHO, NRbCORa, SO₂ORa, SO₂Ra, CONRbSO₂Ra and P(O)(ORa)₂ (where Ra-Rc are each independently hydrogen, lower alkyl or substituted lower alkyl, or Ra-Rc are each independently aryl (C₁₋₇) alkyl, heteroaryl (C₁₋₇) alkyl, aryl or heteroaryl, there being optionally present on the aryl or heteroaryl ring one or more, normally 1 to 3, substituents selected from among Group A defined above. Also, a substituted lower alkyl group may have as substituents 1 to 3 atoms or groups selected from among halogens, nitro and hydroxyl); or R¹ may optionally have on the aforementioned groups (i) to (iii) one or more substituents selected from among those of Cyclic Group G defined as follows. (Cyclic Group G: an optionally substituted 5- or 6-membered heterocycle including 1 to 3 oxygen or nitrogen atoms). R² represents C₁₋₈ alkyl, aryl(C₁₋₇)alkyl, heteroaryl(C₁₋₇)alkyl, or aryl; or R² represents a substituent of Group B defined above, C₁₋₈ alkyl having a substituent of Group B, or C₁₋₈ alkyl having a substituent of Cyclic Group G defined above. R³ represents hydrogen; or R³ represents (i) D(CH₂)₀₋₃^{·}CO, (ii) D^{·}CO^{·}E^{·}CO or (iii) the acyl group D^{·}SO₂^{·}E^{·}CO; or R³ represents the sulfonyl group D(CH₂)₀₋₃^{·}SO₂ or D^{·}CO^{·}E^{·}SO₂ (where the group D represents hydrogen, C₁₋₆ linear, branched or cyclic alkyl, aryl, halogeno lower alkyl, halogeno lower alkoxy, amino, lower alkoxyamino, halogeno lower alkylamino, RbRcN, RbRcN^{·}O, RaO, Ra, RaOCO, RbRcNCO, RaSO₂NRb, RaS or Cyclic Group G defined above, and the group E represents a C₁₋₆ divalent bridging group); or R³ represents the urea group represented by RbRcNCO; or R³ represents the thiourea group represented by RbRcN^{·}CS; or R³ is Ra. X and Y each independently represents a nitrogen or carbon atom, and may be substituted with a group represented by any of Ra-Rc. Z represents a polymethylene group, and the hydrogen atoms on the polymethylene group may be independently substituted with Ra or Rb.]

Of the compounds represented by formula (III), those represented by the following formula (III-I) have been reported to be efficacious when orally administered to canine myocardial infarction models (75th Annual Meeting of the Japanese Pharmacological Society), and their usefulness as chymase inhibitors for the first effective ingredient according to the invention may be expected in circulatory diseases for amelioration of heart failure and improved myocardial infarction prognosis.

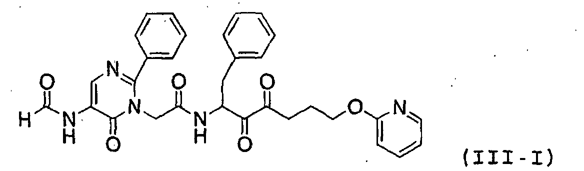

As chymase inhibitors according to the invention there may also be mentioned the compounds disclosed in WO98/18794 which are represented by the following formula (IV): 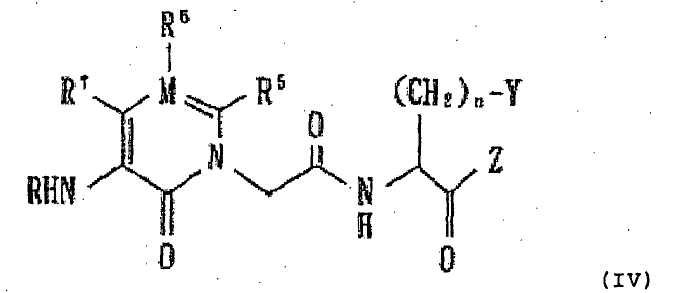 [wherein R represents hydrogen, alkyl, -CHO, -CONH₂, -COR¹, -COOR¹, -CONHOR¹, -CONHR¹, -CONR¹R^{1'}, -CONHSO₂R¹, -COSR¹, -COCOR², -COCOOR², -CONHCOOR², -COCONR³R⁴, -CSXR¹, -SO₂WR¹, -SO₂NR¹R^{1'} or -SO₂E (where R¹ and R^{1'} may be the same or different and each independently represents alkyl, cycloalkyl, cycloalkylalkyl, aryl, arylalkyl, heteroaryl, heteroarylalkyl, a heterocycle or heterocycloalkyl, R², R³ and R⁴ may be the same or different and each independently represents hydrogen, alkyl or arylalkyl, or -NR³R⁴ may together represent a heterocycle, X represents a single bond, -NH-, -O- or -S-, W represents a single bond, -NH-, -NHCO-, -NHCOO or -NHCONH-, and E represents hydroxyl or amino), R⁵, R⁶ and R⁷ may be the same or different and each independently represents hydrogen or alkyl, or one from among R⁵, R⁶ and R⁷ represents aryl, arylalkyl, arylalkenyl, heteroaryl, heteroarylalkyl or heteroarylalkenyl while the others represent hydrogen atoms, M represents a carbon or nitrogen atom, with the proviso that R⁶ is not present when M is a nitrogen atom, Y represents cycloalkyl, aryl or heteroaryl, and Z is a group represented by the following formula (i), (ii) or (iii): 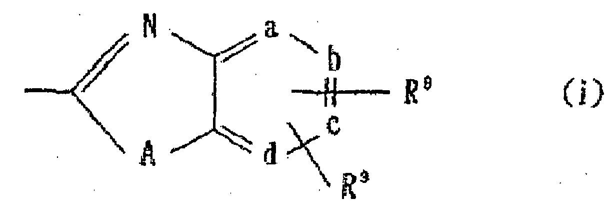 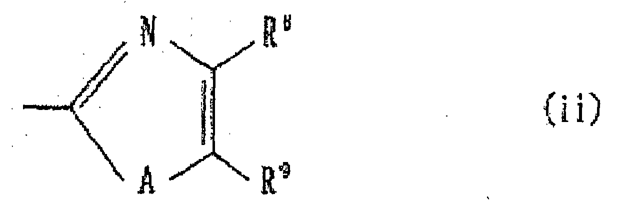 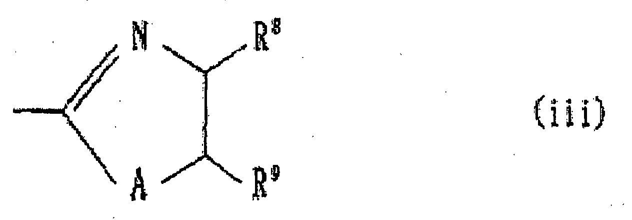 {wherein R⁸ and R⁹ may be the same or different and each independently represents hydrogen, alkyl, aryl, arylalkyl, heteroaryl, heteroarylalkyl, a halogen, trifluoromethyl, cyano, nitro, -NR¹⁰R^{10'}, -NHSO₂R¹⁰, -OR¹⁰, -COOR¹⁰, -CONHSO₂R¹⁰ or -CONR¹⁰R^{10'} (where R¹⁰ and R^{10'} may be the same or different and each independently represents hydrogen, alkyl, cycloalkyl, cycloalkylalkyl, aryl, arylalkyl, heteroaryl, heteroarylalkyl or trifluoromethyl, or -NR¹⁰R^{10'} may together represent a heterocycle), A represents -O-, -S- or -NR¹²- (where R¹² represents hydrogen, alkyl, cycloalkyl or cycloalkylalkyl), and a, b, c and d are all carbon atoms, or one among them represents a nitrogen atom while the others represent carbon atoms}, and n represents 0 or 1. Among the groups mentioned above, the alkyl, cycloalkyl, cycloalkylalkyl, aryl, arylalkyl, arylalkenyl, heteroaryl, heteroarylalkyl, heteroarylalkenyl, heterocyclo and heterocycloalkyl groups may be optionally substituted.]

Of the compounds represented by formula (IV), those represented by the following formula (IV-I) have been reported to exhibit chymase inhibiting activity by oral administration to mouse allergy models (WO00/51640, J. Med. Chem., 2001, Vol.44, p.1286), and they are expected to exhibit effects for circulatory diseases in combination with ACE inhibitors as according to the invention.

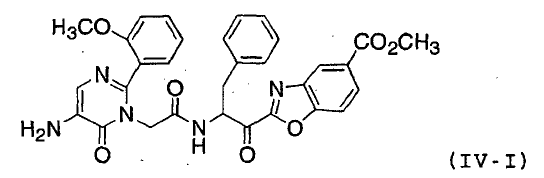

A large number of chymase inhibitors have been reported to date, in addition to those mentioned above. The use of all such compounds as chymase inhibitors according to the invention may be useful in circulatory diseases for treatment of heart failure and improvement of myocardial infarction prognosis.

Examples of previously proposed chymase inhibitors in addition to those mentioned above include the compounds disclosed in WO01/322214, WO02/18378, WO01/122261, WO01/32621, WO02/122595, Japanese Unexamined Patent Publication HEI No. 11-48739, Japanese Unexamined Patent Publication HEI No. 11-1479, Japanese Unexamined Patent Publication HEI No. 10-251239, Japanese Unexamined Patent Publication HEI No. 8-208654, Japanese Unexamined Patent Publication No. 2001-97957, Japanese Unexamined Patent Publication No. 2000-95770, and other publications.

A chymase inhibitor used for the invention powerfully inhibits human chymase activity. Specifically, it has an IC₅₀ of preferably no greater than 1000 nM, more preferably at least 0.01 nM and less than 1000 nM and even more preferably at least 0.05 nM and less than 500 nM. Drug agents having such excellent human chymase inhibiting activity, when used in combination with ACE inhibitors, may be utilized as prophylactic and/or therapeutic agents for clinical use against a variety of circulatory diseases.

There are no particular restrictions on the ACE inhibitors to be used together with chymase inhibitors according to the invention. Numerous ACE inhibitors have already come into clinical use and their usage protocols and safety have been established. As examples there may be mentioned alacepril, imidapril hydrochloride, quinapril hydrochloride, temocapril hydrochloride, delapril hydrochloride, benazepril hydrochloride, captopril, cilazapril, trandolapril, perindopril erbumine, enalapril maleate and lisinopril. All of these ACE inhibitors may be used for the invention, but captopril, temocapril hydrochloride, enalapril maleate and lisinopril are particularly preferred. However, there is no restriction to these so long as the ACE inhibitor used has high safety and efficacy.

### Examples

The invention will now be explained in greater detail by the following examples. It is to be understood, however, that the scope of the invention is not in any sense restricted by these examples.

### Example 1 Preparation of recombinant human mast cell chymase

Recombinant preform human mast cell chymase was prepared according to the method reported by Urata et al. (Journal of Biological Chemistry, Vol.266, p.17173 (1991)). Specifically, Heparin Sepharose (Pharmacia) was used for purification from a culture supernatant of insect cells (Th5) infected with recombinant baculovirus containing cDNA coding for human mast cell chymase. After activating the human mast cell chymase according to the method reported by Murakami et al. (Journal of Biological Chemistry, Vol.270, p.2218 (1995)), it was purified with Heparin Sepharose to obtain the active form human mast cell chymase.

### Example 2 Measurement of recombinant human mast cell chymase enzymatic activity inhibition

After adding 2 µl of a DMSO solution containing a compound of the invention to 50 µl of Buffer A (0.5-3.0 M NaCl, 50 mM Tris-HCl, pH 8.0) containing 1-5 ng of the active form human mast cell chymase obtained in Example 1, there was added 50 µl of Buffer A containing 0.5 mM succinyl-alanyl-histidyl-prolyl-phenylalanylparanitroanilide (Bachem) as a substrate, and reaction was conducted at room temperature for 5 minutes. The time-related change in absorbance at 405 nm was measured to determine the inhibiting activity.

Based on measurement of the.inhibiting activity, Compound Nos. 24, 26, 27, 31, 33, 56, 62, 187, 200, 202, 204, 206, 240 and 24.2 were found to have IC₅₀ = ≥ 1 nM and < 10 nM, while Compound Nos. 2, 6, 9, 10, 20, 22, 43, 45, 60, 62, 92, 128, 164, 182 and 189 were found to have IC₅₀ = ≥ 10 nM and ≤ 100 nM.

Thus, the chymase-inhibiting benzimidazole derivatives used for the invention exhibit powerful chymase inhibiting activity. It was thereby demonstrated that the chymase-inhibiting benzimidazole derivatives are human chymase activity-inhibiting substances which may be clinical useful for prevention and/or treatment of various human chymaseassociated diseases.

### Example 3 Measurement of chymase-inhibiting activity using hamster chymase

Hamster chymase was obtained from a crude product extracted from hamster tongue tissue with an acidic buffer solution and subsequently purified with a Phenyl-Sepharose column (elution: 0.15 M NaCl, 50 mM sodium phosphate, pH 6.5, 50% ethylene glycol) and a Heparin-Cellulofine column (elution: 0.5-2.0 M NaCl). The protein was identified as hamster chymase 1 based on the N-terminal amino acid sequence, and activity inhibition was measured by the same method as for the human chymase. All of the compounds which exhibited inhibiting activity on human chymase exhibited inhibiting activity on the hamster chymase as well. Table 2 shows the inhibition constant for Compound No.33 on hamster chymase, as a representative experiment result.

**Table 2**

| Enzyme | Inhibition constant (nM) |
|---|---|
| Human chymase | 6.2 ± 2.2 |
| Hamster chymase | 30.6 ± 3.8 |
| Mouse chymase | 73.4 ±24.2 |
| Chymotrypsin | > 10000 |
| Trypsin | > 10000 |
| Human tryptase | > 10000 |

These results demonstrated that the human chymase inhibitors used for the invention have high enzyme selectivity and are highly safe as pharmaceutical agents.

### Example 4 Manufacture of tablets

Tablets were manufactured, with each tablet composed of the following composition.

| | |
|---|---|
| Compound (Compound No.33) | 5 mg |
| Temocapril hydrochloride | 1 mg |
| Lactose | 230 mg |
| Potato starch | 80 mg |
| Polyvinylpyrrolidone | 11 mg |
| Magnesium stearate | 5 mg |

The Compound No.33, temocapril hydrochloride, lactose and potato starch were combined, and the mixture was evenly moistened with a 20% ethanol solution containing the polyvinylpyrrolidone, passed through a 20 mesh sieve, dried at 45°C, and passed through a 15 mesh sieve. The obtained granules were mixed with the magnesium stearate and compressed into tablets.

### Example 5 Measurement of blood drug concentrations upon oral administration of chymase inhibitors to hamsters

The chymase inhibitor designated as Compound No.33 was mixed with MF powder feed to a content of 0.1% (w/w) to prepare a hamster feed which was given to hamsters for 5 days. Blood was sampled from the abdominal aorta under ether anesthesia, the serum was extracted, and the serum compound concentrations were measured by high-performance liquid chromatography.

The hamster serum concentration of the parent drug (chymase inhibitor designated as Compound No.33) was 7.89 ±1.09 µM (mean ±S.E., N=4), indicating a sufficient blood drug concentration compared to the inhibiting activity strength.

### Example 6 Chymase inhibitor action in accelerated vascular permeation models by intradermal injection of hamster chymase

Compound No.33 was mixed with powder feed to a content of 0.1% (w/w) to prepare a feed which was given to hamsters (Syrian hamsters, male, 7-week-old) for 5 days. Under ether anesthesia, the hamster backs were shaved and the hamster chymase was intradermally injected (hamster chymase: 1, 0.3, 0.1 µg/site; vehicle: 0.15 M NaCl, 0.1 mg/ml BSA, 10 mM Pi-Na, pH 7.0). Evans Blue (1% (w/v) solution, 5 ml/kg) was intravenously administered just prior to the intradermal injection, and the blue spots produced by chymase intradermal injection were quantified. Specifically, the hamsters were bled to death from the abdominal aorta 30 minutes after intradermal injection, and the skin was sampled using the blueing spots as an index. The sampled skin was placed in a glass test tube, the dye was extracted with 2 ml of EB extract (acetone:0.3% Na₂SO₄ = 7:3), and the dye leakage was measured by colorimetry using a spectrophotometer (620 nm). The results are shown in Fig. 1.

A decrease in dye leakage was found in all of the hamsters of the group which was pre-administered the chymase inhibitor. In addition to Compound No.33, the same experiment was conducted with the same system using.Compound A having the formula shown below (human chymase IC₅₀: 9 nM) as a representative compound for imidazolidine derivatives (WO96/04248) and Compound B having the formula shown below (human chymase IC₅₀: 30 nM) as a representative compound for triazine derivatives (Japanese Unexamined Patent Publication HEI No. 8-208654); however, no clear suppressing action was found against accelerated vascular permeation.

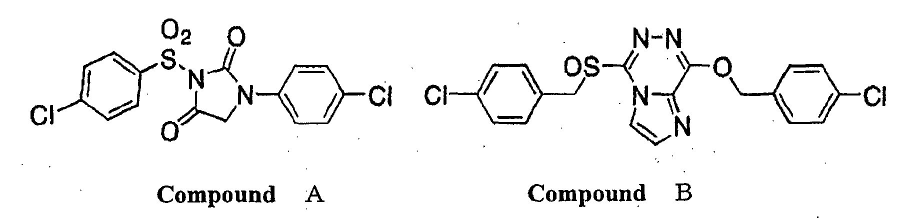

This test verified that compounds of the compound group represented by formula (I) have chymase-inhibiting activity *in vivo* by oral administration, and are therefore preferred as chymase inhibitors.

### Example 7 Combined effect of chymase inhibitor and ACE inhibitor in angiotensin-elicited blood pressure increase models using chymase-overexpressing mice

Human chymase-overexpressing mice (hereinafter abbreviated as "TGM") created by introduction of the human chymase gene with an angiotensin II-producing function were used as the model animals. Specifically, 8- to 10-week-old TGM mice were used, and angiotensin I was continuously infused with a microosmostic pump embedded under the mouse skin (70 ng/kg/min) to create continuous hypertensive mouse models. The continuous infusion of angiotensin I was confirmed to elicit a vasopressor response of about 30 mmHg in the TGM (n=6) with respect to the wild type (n=8), while depressor action was examined in groups administered a chymase inhibitor (Compound No.33, 0.1% diet, n=7), an ACE inhibitor (temocapril hydrochloride, 2.0 mg/kg/day, n=6), an angiotensin receptor blocker (ARB) (Valsartan, 14 mg/kg/day, n=7) and a chymase inhibitor and ACE inhibitor simultaneously (Compound No.33, 0.1% diet, and temocapril hydrochloride, 2.0 mg/kg/day, n=6). The letter "n" indicates the number of individuals. As a result, no notable suppression of vasopressor effect was found in the groups administered the chymase inhibitor alone or the ACE inhibitor alone, as compared to the control group, suggesting that the animal models were analogous to hypertensive patients in which ACE inhibitors exhibit little antihypertensive action. While no suppression of vasopressor effect was found even with administration of the chymase inhibitor alone in these models, the blood pressure values returned to almost normal from the 10th day in the group administered both the ACE inhibitor and the chymase inhibitor, with statistically significant suppression (P <0.05). Continuous blood pressure increase leads to cardiac hypertrophy in these animal models, and no notable suppression of cardiac hypertrophy was found with administration of the ACE inhibitor, angiotensin II receptor blocker (n=7) or chymase inhibitor alone in these models. However, the group administered both the chymase inhibitor and ACE inhibitor showed statistically significant suppression

These experimental results indicated, for the first time, that combined use of an ACE inhibitor and chymase inhibitor may be effective for circulatory diseases for which conventional drugs have proven inefficacious.

### Example 8 Combined effect of chymase inhibitor and ACE inhibitor in hamster myocardial infarction (MI) models

Under pentobarbital anesthesia (50 mg/kg, i.p.), 8-week-old male Syrian hamsters were connected to an electrocardiograph at the four limb extremities and subjected to endotracheal intubation, the chests were opened under respirator control (volume: 10 ml/kg, RR: 60/min), and the left main coronary artery (LAD) was completely ligated with silk thread 2-3 mm from the base. After LAD ligation, a chymase inhibitor (Compound No.33, 0.1% diet), an ACE inhibitor (temocapril hydrochloride, 10 mg/kg/day) or an angiotensin receptor blocker (ARB) (olmesartan, 10 mg/kg/day) was administered alone to one set of groups, while the ACE inhibitor and chymase inhibitor were administered simultaneously to another group, for 35 days, and the survival rates and cardiac functions were analyzed.

The results are shown in Fig. 2. The 30 day survival rates after LAD ligation were 60-70% or greater in the groups administered the chymase inhibitor, ACE inhibitor or angiotensin receptor blocker (ARB), compared to 48% in a placebo-administered group, indicating that these agents are effective alone for improving prognosis after myocardial infarction. In particular, the chymase inhibitor alone produced a survival rate of about 71%, thus confirming an effect equivalent to or surpassing that of the already clinically proven ACE inhibitor and angiotensin receptor blocker. In the group administered both the ACE inhibitor and chymase inhibitor, a statistically significant increase in survival rate was exhibited compared to either of the two agents alone. No deaths occurred in the group administered both the ACE inhibitor and chymase inhibitor during the period from 2 days to 35 days after LAD ligation, and therefore this experiment demonstrated that clinical use of a combination of an ACE inhibitor and chymase inhibitor is drastically effective for treatment of myocardial infarction, as compared to either alone.

### Industrial Applicability

The drug combinations of chymase inhibitors and ACE inhibitors according to the invention, treatment or prophylactic methods using them and treatment agents and methods for circulatory diseases using them, are effective for circulatory diseases, including cardiovascular conditions such as cardiac disease (cardiac hypertrophy, cardiac failure, myocardial infarction, etc.), cerebral apoplexy, vascular injury such as post-PTCA restenosis, arteriosclerosis, renal failure, nephritis and pulmonary hypertension, as well as some other types of hypertension.

## Claims

1. A drug comprising a chymase inhibitor and an ACE inhibitor as effective ingredients.

2. A drug according to claim 1, wherein said chymase inhibitor and said ACE inhibitor are administered simultaneously or separately at different times.

3. A drug according to claim 1 or 2, which is a prophylactic or treatment agent for a circulatory disease.

4. A drug according to claim 3, wherein said circulatory disease is hypertension, cardiac disease, cerebral apoplexy, vascular injury, arteriosclerosis, nephritis or renal failure.

5. A drug according to claim 4, wherein said cardiac disease is cardiac hypertrophy, cardiac failure or myocardial infarction.

6. A drug according to claim 1 or 2 which is an angiotensin II production suppressor.

7. A drug according to any one of claims 1 to 6, wherein said chymase inhibitor and said ACE inhibitor form a mixture.

8. A drug according to any one of claims 1 to 6, wherein said chymase inhibitor and said ACE inhibitor are each independent single agents.

9. A drug according to any one of claims 1 to 6, which is in the form of a kit comprising said chymase inhibitor and said ACE inhibitor.

10. A drug according to any one of claims 1 to 9, wherein said chymase inhibitor is a compound represented by the following formula (I): 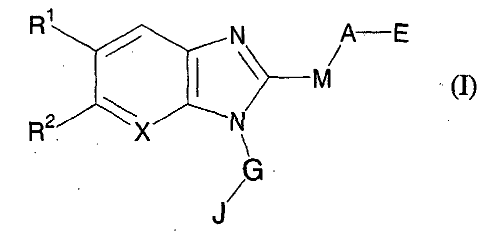 [wherein R¹ and R² may be the same or different and each independently represents a hydrogen atom, a halogen atom, a trihalomethyl group, cyano, hydroxyl, C₁₋₄ alkyl or C₁₋₄ alkoxy, or R¹ and R² may together form -O-CH₂-O-, -O-CH₂CH₂-O-, or -CH₂CH₂CH₂- (where in the case of -O-CH₂-O-, -O-CH₂CH₂-O- or -CH₂CH₂CH₂-, the carbon atoms may be substituted with one or more C₁₋₄ alkyl groups);
A represents a substituted or unsubstituted C₁₋₇ linear, cyclic or branched alkylene or alkenylene group, and may contain therein one or more from among -O-, -S-, -SO₂- and -NR³- (where R³ represents a hydrogen atom or a linear or branched C₁₋₆ alkyl group). Substituents for these groups include halogen atoms, hydroxyl, nitro, cyano, linear or branched C₁₋₆ alkyl, linear or branched C₁₋₆ alkoxy (including 2 or more adjacent ones forming an acetal bond), linear or branched C₁₋₆ alkylthio, linear or branched C₁₋₆ alkylsulfonyl, linear or branched C₁₋₆ acyl, linear or branched C₁₋₆ acylamino, trihalomethyl, trihalomethoxy, phenyl, oxo and phenoxy substituted with one or more halogen atoms. One or more of these substituents may independently substitute at any desired position on the alkylene or alkenylene group;
E represents -COOR³, -SO₃R³, -CONHR³, -SO₂NHR³, tetrazol-5-yl, 5-oxo-1,2,4-oxadiazol-3-yl or 5-oxo-1,2,4-thiadiazol-3-yl (where R³ is the same as defined above);
G represents a substituted or unsubstituted C₁₋₆ linear or branched alkylene group, and may contain therein one or more from among -O-, -S-, -SO₂- and -NR³- (where R³ is the same as defined above, and when such atoms or atomic groups are present they are not bonded directly to the benzimidazole ring). Substituents for these alkylene groups include halogen atoms, hydroxyl, nitro, cyano, linear or branched C₁₋₆ alkyl, linear or branched C₁₋₆ alkoxy (including 2 or more adjacent ones forming an acetal bond), trihalomethyl, trihalomethoxy, phenyl and oxo;
M represents a single bond or -S(O)ₘ- where m is an integer of 0-2;
J represents a substituted or unsubstituted C₄₋₁₀ heteroaryl group having on the ring one or more hetero atoms selected from the group consisting of oxygen, nitrogen and sulfur atoms, with the exception of pyridine. Substituents for these heteroaryl groups include halogen atoms, hydroxyl, nitro, cyano, linear or branched C₁₋₆ alkyl, linear or branched C₁₋₆ alkoxy (including 2 or more adjacent ones forming an acetal bond), linear or branched C₁₋₆ alkylthio, linear or branched C₁₋₆ alkylsulfonyl, linear or branched C₁₋₆ acyl, linear or branched C₁₋₆ acylamino, substituted or unsubstituted anilido, trihalomethyl, trihalomethoxy, phenyl, oxo, COOR³ (where R³ is the same as defined above), or phenoxy substituted with one or more halogen atoms. One or more of these substituents may independently substitute at any desired position on the ring; or J alternatively represents a substituted or unsubstituted C₂₋₆ linear, cyclic or branched alkyl or substituted or unsubstituted C₄₋₁₀ aryl group {Substituents for these groups include halogen atoms, hydroxyl, nitro, cyano, -COOR⁴ (where R⁴ represents a hydrogen atom or C₁₋₄ alkyl group), linear, cyclic or branched C₁₋₆ alkylene, C₁₋₆ linear or branched alkoxy (including 2 or more adjacent ones forming an acetal bond), C₁₋₆ linear or branched alkylthio, C₁₋₆ linear or branched alkylsulfonyl, C₁₋₆ linear or branched alkylsulfinyl, C₁₋₆ acyl, linear or branched C₁₋₆ acylamino, trihalomethyl, trihalomethoxy, phenyl, oxo or phenoxy substituted with one or more halogen atoms. One or more of these substituents may independently substitute at any desired position on the alkylene or aryl group. These substituents may also be in turn substituted with halogen atoms, hydroxyl, nitro, cyano, acyl, trihalomethyl, phenyl, oxo or optionally halogen-substituted phenoxy};
and X represents methine (-CH=) or a nitrogen atom.]

11. A drug according to any one of claims 1 to 9, wherein said chymase inhibitor is a compound represented by the following formula (II): 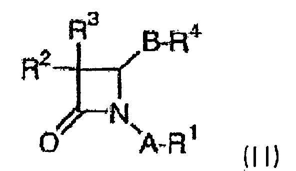 [wherein A is a single bond, -CO-, -COO-, -COCO-, -CONH- or -SO₂-, R¹ is optionally substituted lower alkyl, optionally substituted lower alkenyl, optionally substituted lower alkynyl, optionally substituted cycloalkyl, optionally substituted lower cycloalkenyl or optionally substituted aryl, (and R¹ may be hydrogen when A is a single bond, -CO-, -COCO-, -CONH- or -SO₂-), R² and R³ are each independently hydrogen, a halogen, optionally substituted lower alkyl, optionally substituted lower alkoxycarbonyl, optionally substituted acyl, optionally substituted amino, optionally substituted carbamoyl or optionally substituted aryl, B is a single bond, -S-, -O-, -S-S-, -SO- or -SO₂-, R⁴ is a hydrogen, optionally substituted lower alkyl, optionally substituted aryl, an optionally substituted heterocycle or, when B is a single bond, -S-, -O-, -SO- or -SO₂-, optionally substituted acyl.]

12. A drug according to any one of claims 1 to 9, wherein said chymase inhibitor is a compound represented by the following formula (III): 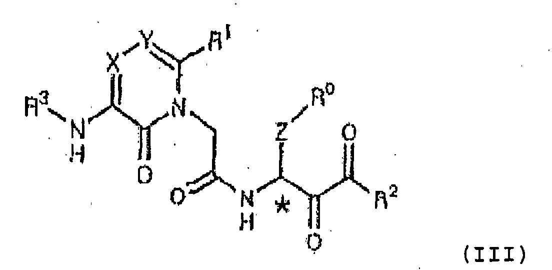 [wherein R⁰ is phenyl, optionally with one or more substituents on the ring selected from among those of Group A defined as follows. (Group A: a halogen, nitro, hydroxyl, lower alkoxy, lower alkyl or halogeno-substituted lower alkyl.) R¹ is (i) aryl, (ii) heteroaryl or (iii) C₁₋₆ linear, branched or cyclic alkyl, optionally each independently having one or more substituents defined according to Group A; R¹ may optionally have on the aforementioned groups (i) to (iii) one or more substituents selected from among those of Group B consisting of ORa, COORa, CONRbRc, NRbRc, NRbCHO, NRbCORa, SO₂ORa, SO₂Ra, CONRbSO₂Ra and P(O)(ORa)₂ (where Ra-Rc are each independently hydrogen, lower alkyl or substituted lower alkyl, or Ra-Rc are each independently aryl (C₁₋₇) alkyl, heteroaryl (C₁₋₇)alkyl, aryl or heteroaryl, there being optionally present on the aryl or heteroaryl ring one or more, normally 1 to 3, substituents selected from among Group A defined above. Also, a substituted lower alkyl group may have as substituents 1 to 3 atoms or groups selected from among halogens, nitro and hydroxyl); or R¹ may optionally have on the aforementioned groups (i) to (iii) one or more substituents selected from among those of Cyclic Group G defined as follows. (Cyclic Group G: an optionally substituted 5- or 6-membered heterocycle including 1 to 3 oxygen or nitrogen atoms). R² represents C₁₋₈ alkyl, aryl(C₁₋₇)alkyl, heteroaryl(C₁₋₇)alkyl, or aryl; or R² represents a substituent of Group B defined above, C₁₋₈ alkyl having a substituent of Group B, or C₁₋₈ alkyl having a substituent of Cyclic Group G defined above. R³ represents hydrogen; or R³ represents (i) D(CH₂)₀₋₃^{·}CO, (ii) D^{·}CO^{·}E^{·}CO or (iii) the acyl group D^{·}SO₂^{·}E^{·}CO; or R³ represents the sulfonyl group D(CH₂)₀₋₃^{·}SO₂ or D^{·}CO^{·}E^{·}SO₂ (where the group D represents hydrogen, C₁₋₆ linear, branched or cyclic alkyl, aryl, halogeno lower alkyl, halogeno lower alkoxy, amino, lower alkoxyamino, halogeno lower alkylamino, RbRcN, RbRcN'O, RaO, Ra, RaOCO, RbRcNCO, RaSO₂NRb, RaS or Cyclic Group G defined above, and the group E represents a C₁₋₆ divalent bridging group); or R³ the urea group represented by RbRcNCO; or R³ represents the thiourea group represented by RbRcN^{·}CS; or R³ is Ra. X and Y each independently represents a nitrogen or carbon atom, and may be substituted with a group represented by any of Ra-Rc. Z represents a polymethylene group, and the hydrogen atoms on the polymethylene group may be independently substituted with Ra or Rb.]

13. A drug according to any one of claims 1 to 9, wherein said chymase inhibitor is a compound represented by the following formula (IV): 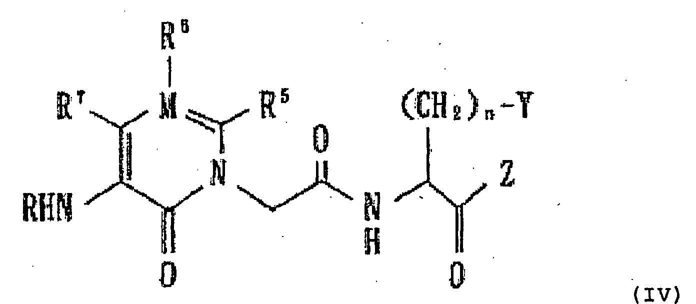 [wherein R represents hydrogen, alkyl, -CHO, -CONH₂, -COR¹, -COOR¹, -CONHOR¹, -CONHR¹, -CONR¹R^{1'}, -CONHSO₂R¹, -COSR¹, -COCOR², -COCOOR², -CONHCOOR², -COCONR³R⁴, -CSXR¹, -SO₂WR¹, -SO₂NR¹R^{1'} or -SO₂E (where R¹ and R^{1'} may be the same or different and each independently represents alkyl, cycloalkyl, cycloalkylalkyl, aryl, arylalkyl, heteroaryl, heteroarylalkyl, a heterocycle or heterocycloalkyl, R², R³ and R⁴ may be the same or different and each independently represents hydrogen, alkyl or arylalkyl, or -NR³R⁴ may together represent a heterocycle, X represents a single bond, -NH-, -O- or -S-, W represents a single bond, -NH-, -NHCO-, -NHCOO or -NHCONH-, and E represents hydroxyl or amino), R⁵, R⁶ and R⁷ may be the same or different and each independently represents hydrogen or alkyl, or one from among R⁵, R⁶ and R⁷ represents aryl, arylalkyl, arylalkenyl, heteroaryl, heteroarylalkyl or heteroarylalkenyl while the others represent hydrogen atoms, M represents a carbon or nitrogen atom, with the proviso that R⁶ is not present when M is a nitrogen atom, Y represents cycloalkyl, aryl or heteroaryl, and Z is a group represented by the following formula (i), (ii) or (iii): 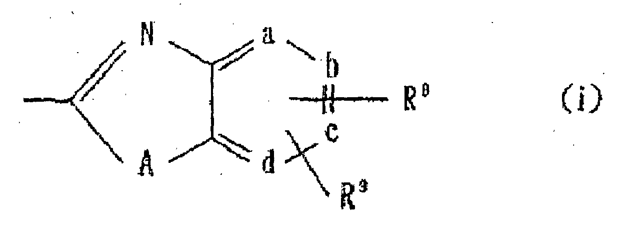 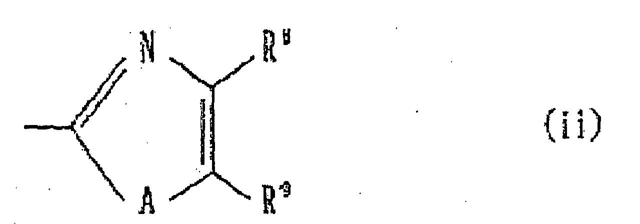 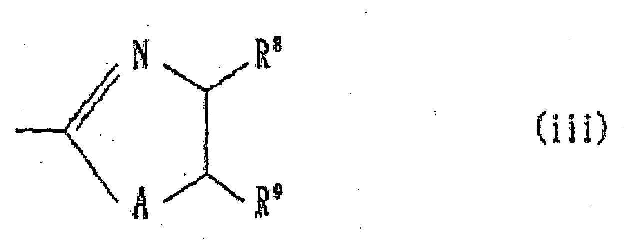 {wherein R⁸ and R⁹ may be the same or different and each independently represents hydrogen, alkyl, aryl, arylalkyl, heteroaryl, heteroarylalkyl, a halogen, trifluoromethyl, cyano, nitro, -NR¹⁰R¹⁰, -NHSO₂R¹⁰, -OR¹⁰, -COOR¹⁰, -CONHSO₂R¹⁰ or -CONR¹⁰R^{10'} (where R¹⁰ and R^{10'} may be the same or different and each independently represents hydrogen, alkyl, cycloalkyl, cycloalkylalkyl, aryl, arylalkyl, heteroaryl, heteroarylalkyl or trifluoromethyl, or -NR¹⁰R^{10'} may together represent a heterocycle), A represents -O-, -S- or -NR¹²- (where R¹² represents hydrogen, alkyl, cycloalkyl or cycloalkylalkyl), and a, b, c and d are all carbon atoms, or one among them represents a nitrogen atom while the others represent carbon atoms}, and n represents 0 or 1. Among the groups mentioned above, the alkyl, cycloalkyl, cycloalkylalkyl, aryl, arylalkyl, arylalkenyl, heteroaryl, heteroarylalkyl, heteroarylalkenyl, heterocyclo and heterocycloalkyl groups may be optionally substituted.]

14. A drug according to any one of claims 1 to 13, wherein said ACE inhibitor is alacepril, imidapril hydrachloride, quinapril hydrochloride, temocapril hydrochloride, delapril hydrochloride, benazepril hydrochloride, captopril, cilazapril, trandolapril, perindopril erbumine, enalapril maleate or lisinopril.

15. A prophylactic or treatment method involving administration of a chymase inhibitor and an ACE inhibitor.

16. A method according to claim 15, wherein said chymase inhibitor and said ACE inhibitor are administered simultaneously or separately at different times.

17. A method according to claim 15 or 16, which is a prophylactic or treatment method for a circulatory disease.

18. A method according to claim 17, wherein said circulatory disease is hypertension, cardiac disease, cerebral apoplexy, vascular injury, arteriosclerosis, nephritis or renal failure.

19. A method according to claim 18, wherein said cardiac disease is cardiac hypertrophy, cardiac failure or myocardial infarction.

20. A method according to claim 15 or 16 which is a method of suppressing angiotensin II production.

21. A method according to any one of claims 15 to 20, wherein said chymase inhibitor is a compound represented by the following formula (I): 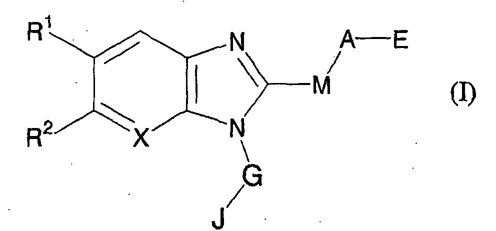 [wherein R¹ and R² may be the same or different and each independently represents a hydrogen atom, a halogen atom, a trihalomethyl group, cyano, hydroxyl, C₁₋₄ alkyl or C₁₋₄ alkoxy, or R¹ and R² may together form -O-CH₂-O-, -O-CH₂CH₂-O-, or -CH₂CH₂CH₂- (where in the case of -O-CH₂-O-, -O-CH₂CH₂-O- or -CH₂CH₂CH₂-, the carbon atoms may be substituted with one or more C₁₋₄ alkyl groups) ;
A represents a substituted or unsubstituted C₁₋₇ linear, cyclic or branched alkylene or alkenylene group, and may contain therein one or more from among -O-, -S-, -SO₂- and -NR³- (where R³ represents a hydrogen atom or a linear or branched C₁₋₆ alkyl group). Substituents for these groups include halogen atoms, hydroxyl, nitro, cyano, linear or branched C₁₋₆ alkyl, linear or branched C₁₋₆ alkoxy (including 2 or more adjacent ones forming an acetal bond), linear or branched C₁₋₆ alkylthio, linear or branched C₁₋₆ alkylsulfonyl, linear or branched C₁₋₆ acyl, linear or branched C₁₋₆ acylamino, trihalomethyl, trihalomethoxy, phenyl, oxo and phenoxy substituted with one or more halogen atoms. One or more of these substituents may independently substitute at any desired position on the alkylene or alkenylene group;
E represents -COOR³, -SO₃R³, -CONHR³, -SO₂NHR³, tetrazol-5-yl, 5-oxo-1,2,4-oxadiazol-3-yl or 5-oxo-1,2,4-thiadiazol-3-yl (where R³ is the same as defined above);
G represents a substituted or unsubstituted C₁₋₆ linear or branched alkylene group, and may contain therein one or more from among -O-, -S-, -SO₂- and -NR³- (where R³ is the same as defined above, and when such atoms or atomic groups are present they are not bonded directly to the benzimidazole ring). Substituents for these alkylene groups include halogen atoms, hydroxyl, nitro, cyano, linear or branched C₁₋₆ alkyl, linear or branched C₁₋₆ alkoxy (including 2 or more adjacent ones forming an acetal bond), trihalomethyl, trihalomethoxy, phenyl and oxo;
M represents a single bond or -S(O)ₘ- where m is an integer of 0-2;
J represents a substituted or unsubstituted C₄₋₁₀ heteroaryl group having on the ring one or more hetero atoms selected from the group consisting of oxygen, nitrogen and sulfur atoms, with the exception of pyridine. Substituents for these heteroaryl groups include halogen atoms, hydroxyl, nitro, cyano, linear or branched C₁₋₆ alkyl, linear or branched C₁₋₆ alkoxy (including 2 or more adjacent ones forming an acetal bond); linear or branched C₁₋₆ alkylthio, linear or branched C₁₋₆ alkylsulfonyl, linear or branched C₁₋₆ acyl, linear or branched C₁₋₆ acylamino, substituted or unsubstituted anilido, trihalomethyl, trihalomethoxy, phenyl, oxo, COOR³ (where R³ is the same as defined above), or phenoxy substituted with one or more halogen atoms. One or more of these substituents may independently substitute at any desired position on the ring; or J alternatively represents a substituted or unsubstituted C₁₋₆ linear, cyclic or branched alkyl or substituted or unsubstituted C₄₋₁₀ aryl group {Substituents for these groups include halogen atoms, hydroxyl, nitro, cyano, -COOR⁴ (where R⁴ represents a hydrogen atom or C₁₋₄ alkyl group), linear, cyclic or branched C₁₋₆ alkylene, C₁₋₆ linear or branched alkoxy (including 2 or more adjacent ones forming an acetal bond), C₁₋₆ linear or branched alkylthio, C₁₋₆ linear or branched alkylsulfonyl, C₁₋₆ linear or branched alkylsulfinyl, C₁₋₆ acyl, linear or branched C₁₋₆ acylamino, trihalomethyl, trihalomethoxy, phenyl, oxo or phenoxy substituted with one or more halogen atoms. One or more of these substituents may independently substitute at any desired position on the alkylene or aryl group. These substituents may also.be in turn substituted with halogen atoms; hydroxyl, nitro, cyano, acyl, trihalomethyl, phenyl, oxo or optionally halogen-substituted phenoxy};
and X represents methine (-CH=) or a nitrogen atom.]

22. A method according to any one of claims 15 to 20, wherein said chymase inhibitor is a compound represented by the following formula (II): 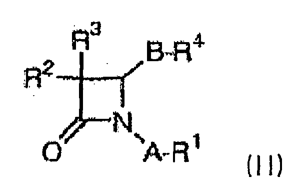 [wherein A is a single bond, -CO-, -COO-, -COCO-, -CONH- or -SO₂-, R¹ is optionally substituted lower alkyl, optionally substituted lower alkenyl, optionally substituted lower alkynyl, optionally substituted cycloalkyl, optionally substituted lower cycloalkenyl or optionally substituted aryl, (and R¹ may be hydrogen when A is a single bond, -CO-, -COCO-, -CONH- or -SO₂-), R² and R³ are each independently hydrogen, a halogen, optionally substituted lower alkyl, optionally substituted lower alkoxycarbonyl, optionally substituted acyl, optionally substituted amino, optionally substituted carbamoyl or optionally substituted aryl, B is a single bond; -S-, -O-, - S-S-, -SO- or -SO₂-, R⁴ is a hydrogen, optionally substituted lower alkyl, optionally substituted aryl, an optionally substituted heterocycle or, when B is a single bond, -S-, -O-, - SO- or -SO₂-, optionally substituted acyl.]

23. A method according to any one of claims 15 to 20, wherein said chymase inhibitor is a compound represented by the following formula (III): 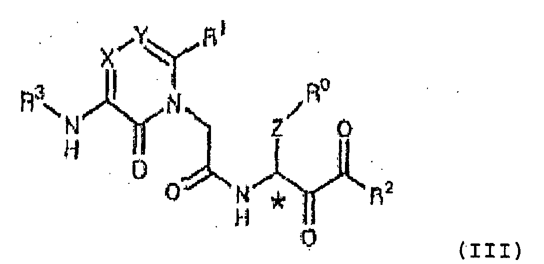 [wherein R⁰ is phenyl, optionally with one or more substituents on the ring selected from among those of Group A defined as follows. (Group A: a halogen, nitro, hydroxyl, lower alkoxy, lower alkyl or halogeno-substituted lower alkyl.) R¹ is (i) aryl, (ii) heteroaryl or (iii) C₁₋₆ linear, branched or cyclic alkyl, optionally each independently having one or more substituents defined according to Group A; R¹ may optionally have on the aforementioned groups (i) to (iii) one or more substituents selected from among those of Group B consisting of ORa, COORa, CONRbRc, NRbRc, NRbCHO, NRbCORa, SO₂ORa, SO₂Ra, CONRbSO₂Ra and P(O)(ORa)₂ (where Ra-Rc are each independently hydrogen, lower alkyl or substituted lower alkyl, or Ra-Rc are each independently aryl(C₁₋₇)alkyl, heteroaryl(C₁₋₇)alkyl, aryl or heteroaryl, there being optionally present on the aryl or heteroaryl ring one or more, normally 1 to 3, substituents selected from among Group A defined above. Also, a substituted lower alkyl group may have as substituents 1 to 3 atoms or groups selected from among halogens, nitro and hydroxyl); or R¹ may optionally have on the aforementioned groups (i) to (iii) one or more substituents selected from among those of Cyclic Group G defined as follows. (Cyclic Group G: an optionally substituted 5- or 6-membered heterocycle including 1 to 3 oxygen or nitrogen atoms). R² represents C₁₋₈ alkyl, aryl(C₁₋₇)alkyl, heteroaryl(C₁₋₇)alkyl, or aryl; or R² represents a substituent of Group B defined above, C₁₋₈ alkyl having a substituent of Group B, or C₁₋₈ alkyl having a substituent of Cyclic Group G defined above. R³ represents hydrogen; or R³ represents (i) D(CH₂)₀₋₃^{·}CO, (ii) D^{·}CO^{·}E^{·}CO or (iii) the acyl group D^{·}SO₂^{·}E^{·}CO; or R³ represents the sulfonyl group D(CH₂)₀₋₃^{·}SO₂ or D^{·}CO^{·}E^{·}SO₂ (where the group D represents hydrogen, C₁₋₆ linear, branched or cyclic alkyl, aryl, halogeno lower alkyl, halogeno lower alkoxy, amino, lower alkoxyamino, halogeno lower alkylamino, RbRcN, RbRcN^{·}O, RaO, Ra, RaOCO, RbRcNCO, RaSO₂NRb, RaS or Cyclic Group G defined above, and the group E represents a C₁₋₆ divalent bridging group); or R³ the urea group represented by RbRcNCO; or R³ represents the thiourea group represented by RbRcN^{·}CS; or R³ is Ra. X and Y each independently represents a nitrogen or carbon atom, and may be substituted with a group represented by any of Ra-Rc. Z represents a polymethylene group, and the hydrogen atoms on the polymethylene group may be independently substituted with Ra or Rb.]

24. A method according to any one of claims 15 to 20, wherein said chymase inhibitor is a compound represented by the following formula (IV): 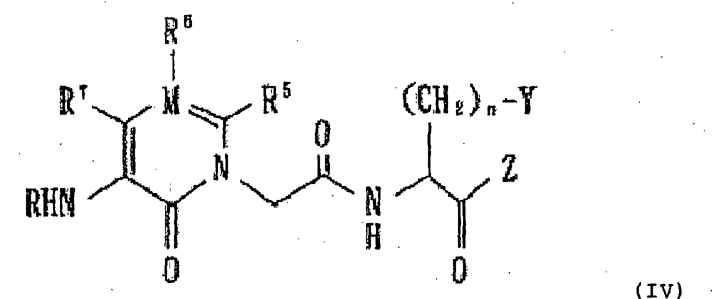 [wherein R represents hydrogen, alkyl, -CHO, -CONH₂, -COR¹, -COOR¹, -CONHOR¹, -CONHR¹, -CONR¹R¹', -CONHSO₂R¹, -COSR¹, -COCOR², -COCOOR², -CONHCOOR², -COCONR³R⁴, -CSXR¹, -SO₂WR¹, -SO₂NR¹R¹' or -SO₂E (where R¹ and R¹' may be the same or different and each independently represents alkyl, cycloalkyl, cycloalkylalkyl, aryl, arylalkyl, heteroaryl, heteroarylalkyl, a heterocycle or heterocycloalkyl, R², R³ and R⁴ may be the same or different and each independently represents hydrogen, alkyl or arylalkyl, or -NR³R⁴ may together represent a heterocycle, X represents a single bond, -NH-, -O- or -S-, W represents a single bond, -NH-, -NHCO-, -NHCOO or -NHCONH-, and E represents hydroxyl or amino), R⁵, R⁶ and R⁷ may be the same or different and each independently represents hydrogen or alkyl, or one from among R⁵, R⁶ and R⁷ represents aryl, arylalkyl, arylalkenyl, heteroaryl, heteroarylalkyl or heteroarylalkenyl while the others represent hydrogen atoms, M represents a carbon or nitrogen atom, with the proviso that R⁶ is not present when M is a nitrogen atom, Y represents cycloalkyl, aryl or heteroaryl, and Z is a group represented by the following formula (i), (ii) or (iii): 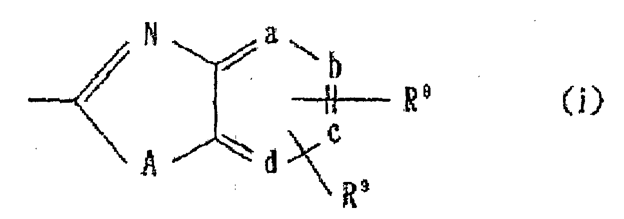 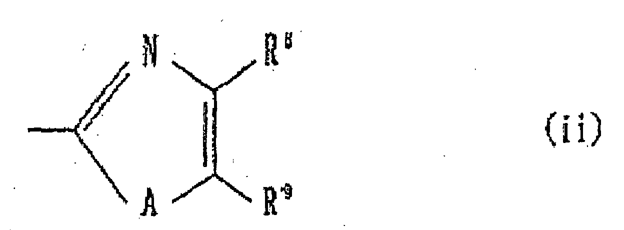 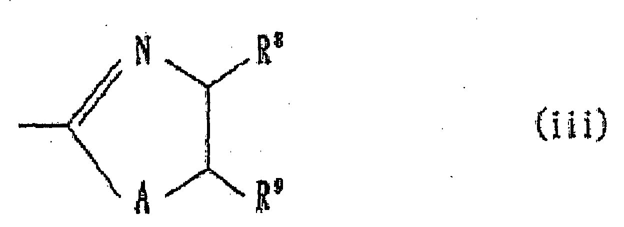 {wherein R⁸ and R⁹ may be the same or different and each independently represents hydrogen, alkyl, aryl, arylalkyl, heteroaryl, heteroarylalkyl, a halogen, trifluoromethyl, cyano, nitro, -NR¹⁰R^{10'}, -NHSO₂R¹⁰, -OR¹⁰, -COOR¹⁰, -CONHSO₂R¹⁰ or -CONR¹⁰R^{10'} (where R¹⁰ and R¹⁰' may be the same or different and each independently represents hydrogen, alkyl, cycloalkyl, cycloalkylalkyl, aryl, arylalkyl, heteroaryl, heteroarylalkyl or trifluoromethyl, or -NR¹⁰R^{10'} may together represent a heterocycle), A represents -O-, -S- or -NR¹²- (where R¹² represents hydrogen, alkyl, cycloalkyl or cycloalkylalkyl), and a, b, c and d are all carbon atoms, or one among them represents a nitrogen atom while the others represent carbon atoms}, and n represents 0 or 1. Among the groups mentioned above, the alkyl, cycloalkyl, cycloalkylalkyl, aryl, arylalkyl, arylalkenyl, heteroaryl, heteroarylalkyl, heteroarylalkenyl, heterocyclo and heterocycloalkyl groups may be optionally substituted.]

25. A method according to any one of claims 15 to 24, wherein said ACE inhibitor is alacepril, imidapril hydrochloride, quinapril hydrochloride, temocapril hydrochloride, delapril hydrochloride, benazepril hydrochloride, captopril, cilazapril, trandolapril, perindopril erbumine, enalapril maleate or lisinopril.

26. A prophylactic or treatment agent for a circulatory disease comprising a chymase inhibitor as an effective ingredient.

27. A prophylactic or treatment agent according to claim 26, wherein said circulatory disease is hypertension, cardiac disease, cerebral apoplexy, vascular injury, arteriosclerosis, nephritis or renal failure.

28. A prophylactic or treatment agent according to claim 27, wherein said cardiac disease is cardiac hypertrophy, cardiac failure or myocardial infarction.

29. An angiotensin II production suppressor comprising a chymase inhibitor as an effective ingredient.

30. A prophylactic agent, treatment agent or suppressor according to any one of claims 26 to 29, wherein said chymase inhibitor is a compound represented by the following formula (I) : 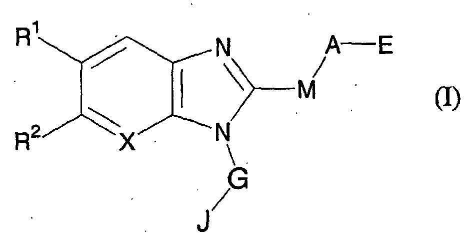 [wherein R¹ and R² may be the same or different and each independently represents a hydrogen atom, a halogen atom, a trihalomethyl group, cyano, hydroxyl, C₁₋₄ alkyl or C₁₋₄ alkoxy, or R¹ and R² may together form -O-CH₂-O-, -O-CH₂CH₂-O-, or -CH₂CH₂CH₂- (where in the case of -O-CH₂-O-, -O-CH₂CH₂-O- or -CH₂CH₂CH₂-, the carbon atoms may be substituted with one or more C₁₋₄ alkyl groups);
A represents a substituted or unsubstituted C₁₋₇ linear, cyclic or branched alkylene or alkenylene group, and may contain therein one or more from among -O-, -S-, -SO₂- and -NR³- (where R³ represents a hydrogen atom or a linear or branched C₁₋₆ alkyl group). Substituents for these groups include halogen atoms, hydroxyl, nitro, cyano, linear or branched C₁₋₆ alkyl, linear or branched C₁₋₆ alkoxy (including 2 or more adjacent ones forming an acetal bond), linear or branched C₁₋₆ alkylthio, linear or branched C₁₋₆ alkylsulfonyl, linear or branched C₁₋₆ acyl, linear or branched C₁₋₆ acylamino, trihalomethyl, trihalomethoxy, phenyl, oxo and phenoxy substituted with one or more halogen atoms. One or more of these substituents may independently substitute at any desired position on the alkylene or alkenylene group;
E represents -COOR³, -SO₃R³, -CONHR³, -SO₂NHR³, tetrazol-5-yl, 5-oxo-1,2,4-oxadiazol-3-yl or 5-oxo-1,2,4-thiadiazol-3-yl (where R³ is the same as defined above);
G represents a substituted or unsubstituted C₁₋₆ linear or branched alkylene group, and may contain therein one or more from among -O-, -S-, -SO₂- and -NR³- (where R³ is the same as defined above, and when such atoms or atomic groups are present they are not bonded directly to the benzimidazole ring). substituents for these alkylene groups include halogen atoms, hydroxyl, nitro, cyano, linear or branched C₁₋₆ alkyl, linear or branched C₁₋₆ alkoxy (including 2 or more adjacent ones forming an acetal bond), trihalomethyl, trihalomethoxy, phenyl and oxo;
M represents a single bond or -S(O)ₘ- where m is an integer of 0-2;
J represents a substituted or unsubstituted C₄₋₁₀ heteroaryl group having on the ring one or more hetero atoms selected from the group consisting of oxygen, nitrogen and sulfur atoms, with the exception of pyridine. Substituents for these heteroaryl groups include halogen atoms, hydroxyl, nitro, cyano, linear or branched C₁₋₆ alkyl, linear or branched C₁₋₆ alkoxy (including 2 or more adjacent ones forming an acetal bond), linear or branched C₁₋₆ alkylthio, linear or branched C₁₋₆ alkylsulfonyl, linear or branched C₁₋₆ acyl, linear or branched C₁₋₆ acylamino, substituted or unsubstituted anilido, trihalomethyl, trihalomethoxy, phenyl, oxo, COOR³ (where R³ is the same as defined above), or phenoxy substituted with one or more halogen atoms. One or more of these substituents may independently substitute at any desired position on the ring; or J alternatively represents a substituted or unsubstituted C₁₋₆ linear, cyclic or branched alkyl or substituted or unsubstituted C₄₋₁₀ aryl group {Substituents for these groups include halogen atoms, hydroxyl, nitro, cyano, -COOR⁴ (where R⁴ represents a hydrogen atom or C₁₋₄ alkyl group), linear, cyclic or branched C₁₋₆ alkylene, C₁₋₆ linear or branched alkoxy (including 2 or more adjacent ones forming an acetal bond), C₁₋₆ linear or branched alkylthio, C₁₋₆ linear or branched alkylsulfonyl, C₁₋₆ linear or branched alkylsulfinyl, C₁₋₆ acyl, linear or branched C₁₋₆ acylamino, trihalomethyl, trihalomethoxy, phenyl, oxo or phenoxy substituted with one or more halogen atoms. One or more of these substituents may independently substitute at any desired position on the alkylene or aryl group. These substituents may also be in turn substituted with halogen atoms, hydroxyl, nitro, cyano, acyl, trihalomethyl, phenyl, oxo or optionally halogen-substituted phenoxy};
and X represents methine (-CH=) or a nitrogen atom.]

31. A prophylactic agent, treatment agent or suppressor according to any one of claims 26 to 29, wherein said chymase inhibitor is a compound represented by the following formula (II) : 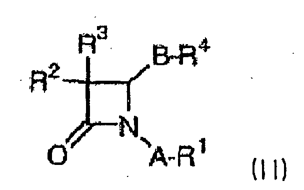 [wherein A is a single bond, -CO-, -COO-, -COCO-, -CONH- or -SO₂-, R¹ is optionally substituted lower alkyl, optionally substituted lower alkenyl, optionally substituted lower alkynyl, optionally substituted cycloalkyl, optionally substituted lower cycloalkenyl or optionally substituted aryl, (and R¹ may be hydrogen when A is a single bond, -CO-, -COCO-, -CONH- or -SO₂-), R² and R³ are each independently hydrogen, a halogen, optionally substituted lower alkyl, optionally substituted lower alkoxycarbonyl, optionally substituted acyl, optionally substituted amino, optionally substituted carbamoyl or optionally substituted aryl, B is a single bond, -S-, -O-, -S-S-, -SO- or -SO₂-, R⁴ is a hydrogen, optionally substituted lower alkyl, optionally substituted aryl, an optionally substituted heterocycle or, when B is a single bond, -S-, -O-, -SO- or -SO₂-, optionally substituted acyl.]

32. A prophylactic agent, treatment agent or suppressor according to any one of claims 26 to 29, wherein said chymase inhibitor is a compound represented by the following formula (III) : 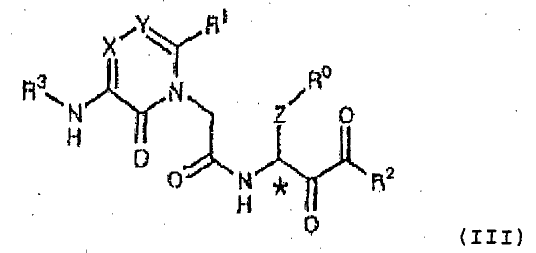 [wherein R⁰ is phenyl, optionally with one or more substituents on the ring selected from among those of Group A defined as follows. (Group A: a halogen, nitro, hydroxyl, lower alkoxy, lower alkyl or halogeno-substituted lower alkyl.) R¹ is (i) aryl, (ii) heteroaryl or (iii) C₁₋₆ linear, branched or cyclic alkyl, optionally each independently having one or more substituents defined according to Group A; R¹ may optionally have on the aforementioned groups (i) to (iii) one or more substituents selected from among those of Group B consisting of ORa, COORa, CONRbRc, NRbRc, NRbCHO, NRbCORa, SO₂ORa, SO₂Ra, CONRbSO₂Ra and P(O)(ORa)₂ (where Ra-Rc are each independently hydrogen, lower alkyl or substituted lower alkyl, or Ra-Rc are each independently aryl (C₁₋₇) alkyl, heteroaryl (C₁₋₇) alkyl, aryl or heteroaryl, there being optionally present on the aryl or heteroaryl ring one or more, normally 1 to 3, substituents selected from among Group A defined above. Also, a substituted lower alkyl group may have as substituents 1 to 3 atoms or groups selected from among halogens, nitro and hydroxyl); or R¹ may optionally have on the aforementioned groups (i) to (iii) one or more substituents selected from among those of Cyclic Group G defined as follows. (Cyclic Group G: an optionally substituted 5- or 6-membered heterocycle including 1 to 3 oxygen or nitrogen atoms). R² represents C₁₋₈ alkyl, aryl(C₁₋₇)alkyl, heteroaryl(C₁₋₇)alkyl, or aryl; or R² represents a substituent of Group B defined above, C₁₋₈ alkyl having a substituent of Group B, or C₁₋₈ alkyl having a substituent of Cyclic Group G defined above. R³ represents hydrogen; or R³ represents (i) D(CH₂)₀₋₃^{·}CO, (ii) D^{·}CO^{·}E^{·}CO or (iii) the acyl group D^{·}SO₂^{·}E^{·}CO; or R³ represents the sulfonyl group D(CH₂)₀₋₃^{.}SO₂ or D^{·}CO^{·}E^{·}SO₂ (where the group D represents hydrogen, C₁₋₆ linear, branched or cyclic alkyl, aryl, halogeno lower alkyl, halogeno lower alkoxy, amino, lower alkoxyamino, halogeno lower alkylamino, RbRcN, RbRcN^{·}O, RaO, Ra, RaOCO, RbRcNCO, RaSO₂NRb, RaS or Cyclic Group G defined above, and the group E represents a C₁₋₆ divalent bridging group); or R³ the urea group represented by RbRcNCO; or R³ represents the thiourea group represented by RbRcN^{·}CS; or R³ is Ra. X and Y each independently represents a nitrogen or carbon atom, and may be substituted with a group represented by any of Ra-Rc. Z represents a polymethylene group, and the hydrogen atoms on the polymethylene group may be independently substituted with Ra or Rb.]

33. A prophylactic agent, treatment agent or suppressor according to any one of claims 26 to 29, wherein said chymase inhibitor is a compound represented by the following formula (IV) : 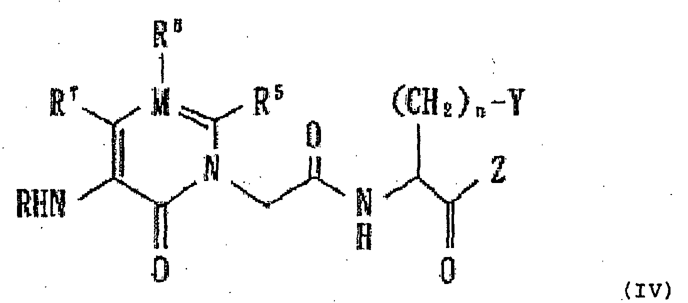 [wherein R represents hydrogen, alkyl, -CHO, -CONH₂, -COR¹, -COOR¹, -CONHOR¹, -CONHR¹, -CONR¹R^{1'}, -CONHSO₂R¹, -COSR¹, -COCOR², -COCOOR², -CONHCOOR², -COCONR³R⁴, -CSXR¹, -SO₂WR¹, -SO₂NR¹R^{1'} or -SO₂E (where R¹ and R¹' may be the same or different and each independently represents alkyl, cycloalkyl, cycloalkylalkyl, aryl, arylalkyl, heteroaryl, heteroarylalkyl, a heterocycle or heterocycloalkyl, R², R³ and R⁴ may be the same or different and each independently represents hydrogen, alkyl or arylalkyl, or -NR³R⁴ may together represent a heterocycle, X represents a single bond, -NH-, -O- or -S-, W represents a single bond, -NH-, -NHCO-, -NHCOO or -NHCONH-, and E represents hydroxyl or amino), R⁵, R⁶ and R⁷ may be the same or different and each independently represents hydrogen or alkyl, or one from among R⁵, R⁶ and R⁷ represents aryl, arylalkyl, arylalkenyl, heteroaryl, heteroarylalkyl or heteroarylalkenyl while the others represent hydrogen atoms, M represents a carbon or nitrogen atom, with the proviso that R⁶ is not present when M is a nitrogen atom, Y represents cycloalkyl, aryl or heteroaryl, and Z is a group represented by the following formula (i), (ii) or (iii): 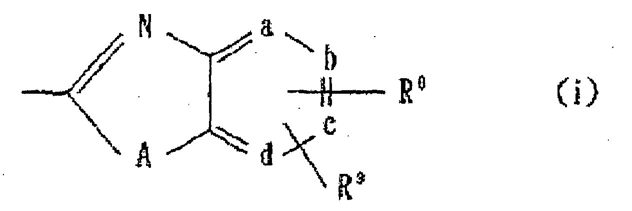 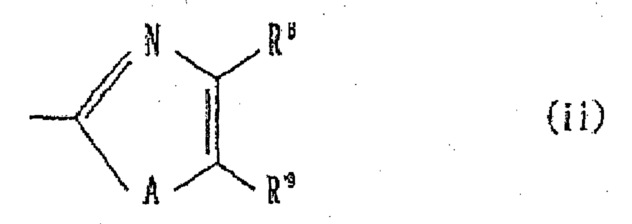 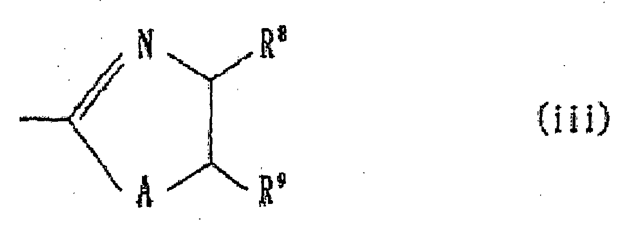 {wherein R⁸ and R⁹ may be the same or different and each independently represents hydrogen, alkyl, aryl, arylalkyl, heteroaryl, heteroarylalkyl, a halogen, trifluoromethyl, cyano, nitro, -NR¹⁰R^{10'}, -NHSO₂R¹⁰, -OR¹⁰, -COOR¹⁰, -CONHSO₂R¹⁰ or -CONR¹⁰R^{10'} (where R¹⁰ and R^{10'} may be the same or different and each independently represents hydrogen, alkyl, cycloalkyl, cycloalkylalkyl, aryl, arylalkyl, heteroaryl, heteroarylalkyl or trifluoromethyl, or -NR¹⁰R^{10'} may together represent a heterocycle), A represents -O-, -S- or -NR¹²- (where R¹² represents hydrogen, alkyl, cycloalkyl or cycloalkylalkyl), and a, b, c and d are all carbon atoms, or one among them represents a nitrogen atom while the others represent carbon atoms},. and n represents 0 or 1. Among the groups mentioned above, the alkyl, cycloalkyl, cycloalkylalkyl, aryl, arylalkyl, arylalkenyl, heteroaryl, heteroarylalkyl, heteroarylalkenyl, heterocyclo and heterocycloalkyl groups may be optionally substituted.]
